# EUROPÄISCHE PATENTSCHRIFT

(11) **EP 0 943 614 B1**
(45) Veröffentlichungstag und Bekanntmachung des Hinweises auf die Patenterteilung: **12.12.2001**
(21) Anmeldenummer: 99101073.7
(22) Anmeldetag: 25.01.1999
(51) Int. Cl.: C07D 333/20, C07D 307/52, C07D 207/32, A61K 7/13, C07D 207/335, C07D 333/22, C07D 333/28, C07D 333/42, C07D 333/44

(54) **Diaminobenzol-Derivate enthaltende Färbemittel sowie die Diaminobenzol-Derivate**
Dyeing agents comprising diaminobenzene derivatives and the diaminobenzene derivatives
Colorants contenant des dérivés de diaminobenzène et dérivés de diaminobenzène

(30) Priorität: 19.03.1998 DE 19812059
(43) Veröffentlichungstag der Anmeldung: 22.09.1999
(73) Patentinhaber: Wella Aktiengesellschaft, 64295 Darmstadt (DE)
(72) Erfinder: Braun, Hans-Jürgen, Dr., 3182 Überstorf (CH); Chassot, Laurent, Dr., 1724 Praroman (CH)

(56) Entgegenhaltungen:
- EP-A- 0 604 353
- DE-A- 2 518 393
- US-A- 5 122 611
- US-A- 5 261 926

## Beschreibung

Die Erfindung betrifft Mittel zum Färben von Keratinfasem auf der Basis einer Entwicklersubstanz-Kupplersubstanz-Kombination, welche als Entwicklersubstanz p-Diaminobenzol-Derivate enthalten, sowie neue p-Diaminobenzol-Derivate.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol und 1,4-Diaminobenzol eingesetzt, während als Kupplersubstanzen beispielsweise Resorcin, 4-Chlorresorcin, 1-Naphthol, 3-Aminophenol und Derivate des m-Phenylendiamins zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

In der EP 0 604 353 A1 wird ein Verfahren zur Herstellung von Benzimidazolen beschrieben, bei dem als Zwischenprodukt u.a. ein 4-N,N-Dimethylamino-3-(2-furyl)-anilin eingesetzt wird. Die Eignung dieser Verbindung als Haarfarbstoff wird dort jedoch nicht beschrieben.
In der US 5 122 611 A wird ein Verfahren zur Herstellung von für den Einsatz in fotografischen, lichtempfindlichen oder wärmeübertragbaren Materialien geeigneten Azomethinen oder Indoanilinen beschrieben, bei dem p-Phenylendiamine mit aktiven Methylen- oder Methinverbindungen zu den Zielverbindungen umgesetzt werden.
Aus der DE 25 18 393 A1 sind Haarfärbemittel bekannt, welche als Entwicklersubstanzen symmetrische Diamino-dihydroxy-diphenyle, symmetrische Diamino-dihydroxy-diphenylether, symmetrische Tetraamino-diphenyle beziehungsweise symmetrische Tetraaminodiphenylether enthalten.
Aus der US 5 261 926 A ist ein Verfahren zum Färben von Keratinfasem bekannt, bei dem zur Färbung eine Kombination aus bestimmten Isatinderivaten und bestimmten substituierten Anilinen oder Bisphenylalkylendiaminen verwendet wird.

Mit den derzeit bekannten und eingesetzten Färbemitteln ist es jedoch nicht möglich, die vorgenannten Anforderungen in allen Punkten zu erfüllen.

Es besteht daher weiterhin ein Bedürfnis nach neuen Entwicklersubstanzen, welche die vorgenannten Anforderung in besonderem Masse erfüllen.

Hierzu wurde nun überraschenderweise gefunden, daß bestimmte p-Diaminobenzol-Derivate gemäß der allgemeinen Formel (I) die an Entwicklersubstanzen gestellten Anforderungen in besonders hohem Maße erfüllen. So werden unter Verwendung dieser Entwicklersubstanzen mit den meisten bekannten Kupplersubstanzen farbstarke Farbnuancen erhalten, die außerordentlich lichtecht und waschecht sind.

Gegenstand der vorliegende Erfindung sind daher Mittel zum oxidativen Färben von Keratinfasern, wie zum Beispiel Haaren, Pelzen, Federn oder Wolle, insbesondere menschlichen Haaren, auf der Basis einer Entwicklersubstanz-Kupplersubstanz-Kombination, welche als Entwicklersubstanz mindestens ein Diaminobenzol-Derivat der Formel (I) 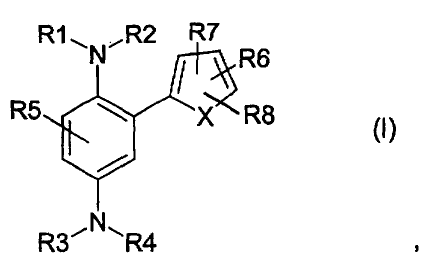 worin
**X** gleich Sauerstoff, Schwefel, Selen oder N-R9 ist;
**R1,R2,R3 und R4** unabhängig voneinander Wasserstoff, eine C₁-C₆-Alkylgruppe, eine C₁-C₄-Hydroxyalkylgruppe, eine C₂-C₄ Dihydroxyalkylgruppe oder eine C₁-C₄-Alkoxy-(C₁-C₂)-alkylgruppe darstellen oder R1und R2 beziehungsweise R3 und R4 einen viergliedrigen bis achtgliedrigen aliphatischen Ring bilden, wobei mindestens 2 der Reste R1 bis R4 Wasserstoff darstellen;
**R5** gleich Wasserstoff, einem Halogenatom, einer C₁-C₄-Alkylgruppe, einer C₁-C₄-Hydroxyalkylgruppe oder einer C₁-C₄-Alkoxygruppe ist;
**R6 und R8** unabhänging voneinander gleich Wasserstoff, einer Hydroxygruppe, einem Halogenatom, einer Cyanogruppe, einer C₁-C₄-Alkoxygruppe, einer C₁-C₆ -Alkylgruppe, einer C₁-C₄-Alkylthioethergruppe, einer Mercaptogruppe, einer Nitrogruppe, einer Aminogruppe, einer Alkylaminogruppe, einer Dialkylaminogruppe, einer -C(O)H-Gruppe, einer -C(O)CH₃-Gruppe, einer-C(O)CF₃-Gruppe, einer -Si(CH₃)₃-Gruppe, einer C₁-C₄-Hydroxyalkylgruppe, einer C₃-C₄-Dihydroxyalkylgruppe, einer -CH=CHR10-Gruppe, einer -(CH₂)ₚ-CO₂R11-Gruppe oder einer -(CH₂)ₚ-R12-Gruppe mit p=1,2,3 oder 4, einer -C(R13)=NR14-Gruppe oder einer C(R16)H-NR17R18-Gruppe sind;
**R7** gleich Wasserstoff, einem Halogenatom, einer C₁-C₆ Alkylgruppe oder einer -C(O)H-Gruppe ist;
**R9** gleich Wasserstoff, einer C₁-C₆-Alkylgruppe, einer C₁-C₄-Hydroxyalkylgruppe, einer Phenylgruppe oder einer Acetylgruppe ist;
**R10** gleich Wasserstoff, einer Hydroxygruppe, einer Nitrogruppe, einer Aminogruppe, einer -CO₂R11-Gruppe oder einer -C(O)CH₃-Gruppe ist;
**R11, R13 und R16** unabhänging voneinander gleich Wasserstoff oder einer C₁-C₄-Alkylgruppe sind;
**R12** gleich einer Aminogruppe oder einer Nitrilgruppe ist;
**R14, R17 und R18** unabhängig voneinander gleich Wasserstoff, einer Hydroxygruppe, einer C₁-C₄-Alkylgruppe, einer C₁-C₄-Hydroxyalkylgruppe, einer C₃-C₄-Dihydroxyalkylgruppe oder einem Rest der Formel (II) sind 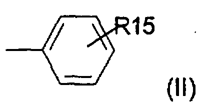 und
**R15** gleich Wasserstoff, einer Aminogruppe oder einer Hydroxygruppe ist, oder deren physiologisch verträgliche, wasserlösliche Salze enthalten.

Als Verbindungen der Formel (I) können beispielweise die folgenden Verbindungen genannt werden:
2,5-Diamino-(2-thienyl)benzol, 2-Amino-5-methylamino-(2-thienyl)benzol, 5-Amino-2-methylamino-(2-thienyl)benzol, 2-Amino-5-dimethylamino-(2-thienyl)benzol, 5-Amino-2-dimethylamino-(2-thienyl)benzol, 2-Amino-5-(2-hydroxyethyl)amino-(2-thienyl)benzol, 5-Amino-2-(2-hydroxyethyl)-amino-(2-thienyl)benzol, 2-Amino-5-di(2-hydroxyethyl)amino-(2-thienyl)benzol, 5-Amino-2-di(2-hydroxyethyl)amino-(2-thienyl)benzol, 2-Amino-5-(2-hydroxyethyl)methylamino-(2-thienyl)benzol, 5-Amino-2-(2-hydroxyethyl)methylamino-(2-thienyl)benzol, 2-Amino-5-(2,3-dihydroxypropyl)-amino-(2-thienyl)benzol, 5-Amino-2-(2,3-dihydroxypropyl)amino-(2-thienyl)benzol, 2-Amino-5-di(2,3-dihydroxypropyl)amino-(2-thienyl)benzol, 5-Amino-2-di(2,3-dihydroxypropyl)amino-(2-thienyl)benzol, 2-Amino-5-(2,3-dihydroxypropyl)methylamino-(2-thienyl)benzol, 5-Amino-2-(2,3-dihydroxypropyl)methylamino-(2-thienyl)benzol, 2-Amino-5-(2-methoxyethyl)amino-(2-thienyl)benzol, 5-Amino-2-(2-methoxyethyl)-amino-(2-thienyl)benzol, 2-Amino-5-di(2-methoxyethyl)amino-(2-thienyl)benzol, 5-Amino-2-di(2-methoxyethyl)amino-(2-thienyl)benzol, 2-Amino-5-(2-methoxyethyl)methylamino-(2-thienyl)benzol, 5-Amino-2-(2-methoxyethyl)methylamino-(2-thienyl)benzol, 2-Amino-5-methylamino-(2-furyl)benzol, 5-Amino-2-methylamino-(2-furyl)benzol, 2-Amino-5-dimethylamino-(2-furyl)benzol, 5-Amino-2-dimethylamino-(2-furyl)benzol, 2-Amino-5-(2-hydroxyethyl)amino-(2-furyl)benzol, 5-Amino-2-(2-hydroxyethyl)amino-(2-furyl)benzol, 2-Amino-5-di(2-hydroxyethyl)amino-(2-furyl)benzol, 5-Amino-2-di(2-hydroxyethyl)amino-(2-furyl)-benzol, 2-Amino-5-(2-hydroxyethyl)methylamino-(2-furyl)benzol, 5-Amino-2-(2-hydroxyethyl)methylamino-(2-furyl)benzol, 2-Amino-5-(2,3-dihydroxypropyl)amino-(2-furyl)benzof, 5-Amino-2-(2,3-dihydroxypropyl)amino-(2-furyl)benzol, 2-Amino-5-di(2,3-dihydroxypropyl)amino-(2-furyl)benzol, 5-Amino-2-di(2,3-dihydroxypropyl)amino-(2-furyl)-benzol, 2-Amino-5-(2,3-dihydroxypropyl)methylamino-(2-furyl)benzol, 5-Amino-2-(2,3-dihydroxypropyl)methylamino-(2-furyl)benzol, 2-Amino-5-(2-methoxyethyl)amino-(2-furyl)benzol, 5-Amino-2-(2-methoxyethyl)amino-(2-furyl)benzol, 2-Amino-5-di(2-methoxyethyl)amino-(2-furyl)benzol, 5-Amino-2-di(2-methoxyethyl)amino-(2-furyl)benzol, 2-Amino-5-(2-methoxyethyl)methylamino-(2-furyl)benzol, 5-Amino-2-(2-methoxyethyl)methylamino-(2-furyl)benzol, 2-Amino-5-methylamino(pyrrol-2-yl)benzol, 5-Amino-2-methylamino-(pyrrol-2-yl)benzol, 2-Amino-5-dimethylamino-(pyrrol-2-yl)benzol, 5-Amino-2-dimethylamino-(pyrrol-2-yl)benzol, 2-Amino-5-(2-hydroxyethyl)amino-(pyrrol-2-yl)benzol, 5-Amino-2-(2-hydroxyethyl)amino-(pyrrol-2-yl)benzol, 2-Amino-5-di(2-hydroxyethyl)amino-(pyrrol-2-yl)benzol, 5-Amino-2-di(2-hydroxyethyl)amino-(pyrrol-2-yl)benzol, 2-Amino-5-(2-hydroxyethyl)methylamino-(pyrrol-2-yl)benzol, 5-Amino-2-(2-hydroxyethyl)methylamino-(pyrrol-2-yl)benzol, 2-Amino-5-(2,3-dihydroxypropyl)amino-(pyrrol-2-yl)benzol, 5-Amino-2-(2,3-dihydroxypropyl)amino-(pyrrol-2-yl)benzol, 2-Amino-5-di(2,3-dihydroxypropyl)amino-(pyrrol-2-yl)benzol, 5-Amino-2-di(2,3-dihydroxypropyl)amino-(pyrrol-2-yl)benzol, 2-Amino-5-(2,3-dihydroxypropyl)methylamino-(pyrrol-2-yl)benzol, 5-Amino-2-(2,3-dihydroxypropyl)methylamino-(pyrrol-2-yl)benzol, 2-Amino-5-(2-methoxyethyl)amino-(pyrrol-2-yl)benzol, 5-Amino-2-(2-methoxyethyl)amino-(pyrrol-2-yl)benzol, 2-Amino-5-di(2-methoxyethyl)amino(pyrrol-2-yl)benzol, 5-Amino-2-di(2-methoxyethyl)amino-(pyrrol-2-yl)benzol, 2-Amino-5-(2-methoxyethyl)methylamino-(pyrrol-2-yl)benzol, 5-Amino-2-(2-methoxyethyl)methylamino-(pyrrol-2-yl)benzol, 2-Amino-5-methylamino-(1 -methyl-1 H-pyrrol-2-yl)benzol, 5-Amino-2-methylamino(1-methyl-1 H-pyrrol-2-yl)benzol, 2-Amino-5-dimethylamino-(1-methyl-1H-pyrrol-2-yl)benzol, 5-Amino-2-dimethylamino-(1-methyl-1H-pyrrol-2-yl)benzol, 2-Amino-5-(2-hydroxyethyl)amino-(1-methyl-1H-pyrrol-2-yl)benzol, 5-Amino-2-(2-hydroxyethyl)amino-(1-methyl-1H-pyrrol-2-yl)benzol, 2-Amino-5-di(2-hydroxyethyl)amino-(1-methyl-1H-pyrrol-2-yl)benzol, 5-Amino-2-di(2-hydroxyethyl)amino-(1-methyl-1H-pyrrol-2-yl)benzol, 2-Amino-5-(2-hydroxyethyl)methylamino-(1-methyl-1H-pyrrol-2-yl)benzol, 5-Amino-2-(2-hydroxyethyl)methylamino-(1-methyl-1H-pyrrol-2-yl)benzol, 2-Amino-5-(2,3-dihydroxypropyl)amino-(1-methyl-1H-pyrrol-2-yl)benzol, 5-Amino-2-(2,3-dihydroxypropyl)amino-(1 -methyl-1H-pyrrol-2-yl)benzol, 2-Amino-5-di(2,3-dihydroxypropyl)amino-(1-methyl-1H-pyrrol-2-yl)benzol, 5-Amino-2-di(2,3-dihydroxypropyl)amino-(1-methyl-1H-pyrrol-2-yl)benzol, 2-Amino-5-(2,3-dihydroxypropyl)methylamino-(1-methyl-1H-pyrrol-2-yl)benzol, 5-Amino-2-(2,3-dihydroxypropyl)methylamino-(1-methyl-1H-pyrrol-2-yl)benzol, 2-Amino-5-(2-methoxyethyl)amino-(1-methyl-1H-pyrrol-2-yl)benzol, 5-Amino-2-(2-methoxyethyl)amino-(1-methyl-1H-pyrrol-2-yl)benzol, 2-Amino-5-di(2-methoxyethyl)amino-(1-methyl-1H-pyrrol-2-yl)benzol, 5-Amino-2-di(2-methoxyethyl)amino-(1-methyl-1H-pyrröl-2-yl)benzol, 2-Amino-5-(2-methoxyethyl)methylamino-(1-methyl-1H-pyrrol-2-yl)benzol, 5-Amino-2-(2-methoxyethyl)methylamino-(1-methyl-1H-pyrrol-2-yl)benzol, 2,5-Diamino-3-methyl-(2-thienyl)benzol, 2-Amino-5-di(2-hydroxyethyl)amino-3-methyl-(2-thienyl)benzol, 5-Amino-2-di(2-hydroxyethyl)amino-3-methyl-(2-thienyl)benzol, 2,5-Diamino-3-chlor-(2-thienyl)benzol, 2-Amino-5-di(2-hydroxyethyl)amino-3-chlor-(2-thienyl)benzol, 5-Amino-2-di(2-hydroxyethyl)amino-3-chlor-(2-thienyl)benzol, 2,5-Diamino-4-methyl-(2-thienyl)benzol, 2-Amino-5-di(2-hydroxyethyl)amino-4-methyl-(2-thienyl)benzol, 5-Amino-2-di(2-hydroxyethyl)amino-4-methyl-(2-thienyl)benzol, 2,5-Diamino-4-chlor-(2-thienyl)benzol, 2-Amino-5-di(2-hydroxyethyl)amino-4-chlor-(2-thienyl)benzol, 5-Amino-2-di(2-hydroxyethyl)amino-4-chlor-(2-thienyl)benzol, 2,5-Diamino-5-methyl-(2-thienyl)benzol, 2-Amino-5-di(2-hydroxyethyl)amino-5-methyl-(2-thienyl)benzol, 5-Amino-2-di(2-hydroxyethyl)amino-5-methyl-(2-thienyl)benzol, 2,5-Diamino-5-chlor-(2-thienyl)benzol, 2-Amino-5-di(2-hydroxyethyl)amino-5-chlor-(2-thienyl)benzol, 5-Amino-2-di(2-hydroxyethyl)amino-5-chlor-(2-thienyl)benzol, 2,5-Diamino-3-methyl-(2-furyl)benzol, 2-Amino-5-di(2-hydroxyethyl)amino-3-methyl-(2-furyl)benzol, 5-Amino-2-di(2-hydroxyethyl)amino-3-methyl-(2-furyl)benzol, 2,5-Diamino-3-chlor-(2-furyl)benzol, 2-Amino-5-di(2-hydroxyethyl)amino-3-chlor-(2-furyl)benzol, 5-Amino-2-di(2-hydroxyethyl)amino-3-chlor-(2-furyl)benzol, 2,5-Diamino-4-methyl-(2-furyl)benzol, 2-Amino-5-di(2-hydroxyethyl)amino-4-methyl-(2-furyl)benzol, 5-Amino-2-di(2-hydroxyethyl)amino-4-methyl-(2-furyl)benzol, 2,5-Diamino-4-Chlor-(2-furyl)benzol, 2-Amino-5-di(2-hydroxyethyl)-amino-4-chlor-(2-furyl)benzol, 5-Amino-2-di(2-hydroxyethyl)amino-4-chlor-(2-furyl)benzol, 2,5-Diamino-5-methyl-(2-furyl)benzol, 2-Amino-5-di(2-hydroxyethyl)amino-5-methyl-(2-furyl)benzol, 5-Amino-2-di(2-hydroxyethyl)amino-5-methyl-(2-furyl)benzol, 2,5-Diamino-5-chlor-(2-furyl)benzol, 2-Amino-5-di(2-hydroxyethyl)amino-5-chlor-(2-furyl)benzol, 5-Amino-2-di(2-hydroxyethyl)amino-5-chlor-(2-furyl)benzol, 2,5-Diamino-3-methyl-(pyrrol-2-yl)benzol, 2-Amino-5-di(2-hydroxyethyl)amino-3-methyl-(pyrrol-2-yl)benzol, 5-Amino-2-di(2-hydroxyethyl)amino-3-methyl-(pyrrol-2-yl)benzol, 2,5-Diamino-3-chlor-(pyrrol-2-yl)benzol, 2-Amino-5-di(2-hydroxyethyl)amino-3-chlor-(pyrrol-2-yl)benzol, 5-Amino-2-di(2-hydroxyethyl)amino-3-chlor-(pyrrol-2-yl)benzol, 2,5-Diamino-4-methyl-(pyrrol-2-yl)benzol, 2-Amino-5-di(2-hydroxyethyl)-amino-4-methyl-(pyrrol-2-yl)benzol, 5-Amino-2-di(2-hydroxyethyl)amino-4-methyl-(pyrrol-2-yl)benzol, 2,5-Diamino-4-chlor-(pyrrol-2-yl)benzol, 2-Amino-5-di(2-hydroxyethyl)amino-4-chlor-(pyrrol-2-yl)benzol, 5-Amino-2-di(2-hydroxyethyl)amino-4-chlor-(pyrrol-2-yl)benzol, 2,5-Diamino-5-methyl(pyrrol-2-yl)benzol, 2-Amino-5-di(2-hydroxyethyl)amino-5-methyl-(pyrrol-2-yl)benzol, 5-Amino-2-di(2-hydroxyethyl)amino-5-methyl-(pyrrol-2-yl)benzol, 2,5-Diamino-5-chlor-(pyrrol-2-yl)benzol, 2-Amino-5-di(2-hydroxyethyl)amino-5-chlor-(pyrrol-2-yl)benzol, 5-Amino-2-di(2-hydroxyethyl)amino-5-chlor-(pyrrol-2-yl)benzol, 2,5-Diamino-3-methyl-(1-methyl-1H-pyrrol-2-yl)-benzol, 2-Amino-5-di(2-hydroxyethyl)amino-3-methyl-(1-methyl-1H-pyrrol-2-yl)benzol, 5-Amino-2-di(2-hydroxyethyl)amino-3-methyl-(1-methyl-1H-pyrrol-2-yl)benzol, 2,5-Diamino-3-chlor-(1-methyl-1H-pyrrol-2-yl)benzol, 2-Amino-5-di(2-hydroxyethyl)amino-3-chlor-(1-methyl-1H-pyrrol-2-yl)benzol, 5-Amino-2-di(2-hydroxyethyl)amino-3-chlor-(1-methyl-1H-pyrrol-2-yl)benzol, 2,5-Diamino-4-methyl-(1-methyl-1H-pyrrol-2-yl)benzol, 2-Amino-5-di(2-hydroxyethyl)amino-4-methyl-(1-methyl-1H-pyrrol-2-yl)benzol, 5-Amino-2-di(2-hydroxyethyl)amino-4-methyl-(1-methyl-1H-pyrrol-2-yl)benzol, 2,5-Diamino-4-chlor-(1 -methyl-1 H-pyrrol-2-yl)benzol, 2-Amino-5-di(2-hydroxyethyl)amino-4-chlor-(1-methyl-1H-pyrrol-2-yl)benzol, 5-Amino-2-di(2-hydroxyethyl)amino-4-chlor-(1-methyl-1H-pyrrol-2-yl)benzol, 2,5-Diamino-5-methyl-(1-methyl-1 H-pyrrol-2-yl)benzol, 2-Amino-5-di(2-hydroxyethyl)amino-5-methyl-(1-methyl-1 H-pyrrol-2-yl)benzol, 5-Amino-2-di(2-hydroxyethyl)amino-5-methyl-(1-methyl-1 H-pyrrol-2-yl)benzol, 2,5-Diamino-5-chlor-(1-methyl-1 H-pyrrol-2-yl)benzol, 2-Amino-5-di(2-hydroxyethyl)amino-5-chlor-(1-methyl-1H-pyrrol-2-yl)benzol, 5-Amino-2-di(2-hydroxyethyl)amino-5-chlor-(1-methyl-1H-pyrrol-2-yl)benzol, 2,5-Diamino-(3-methyl-2-thienyl)benzol, 2-Amino-5-di(2-hydroxyethyl)amino-(3-methyl-2-thienyl)benzol, 5-Amino-2-di(2-hydroxyethyl)amino(3-methyl-2-thienyl)benzol, 2,5-Diamino-(3-methyl-2-furyl)benzol, 2-Amino-5-di(2-hydroxyethyl)amino-(3-methyl-2-furyl)benzol, 5-Amino-2-di(2-hydroxyethyl)amino-(3-methyl-2-furyl)benzol, 2,5-Diamino-(4-methyl-2-thienyl)benzol, 2-Amino-5-di(2-hydroxyethyl)amino-(4-methyl-2-thienyl)benzol, 5-Amino-2-di(2-hydroxyethyl)amino-(4-methyl-2-thienyl)benzol, 2,5-Diamino-(4-methyl-2-furyl)benzol, 2-Amino-5-di(2-hydroxyethyl)amino-(4-methyl-2-furyl)benzol, 5-Amino-2-di(2-hydroxyethyl)amino-(4-methyl-2-furyl)benzol, 2,5-Diamino-(5-methyl-2-thienyl)benzol, 2-Amino-5-di(2-hydroxyethyl)amino-(5-methyl-2-thienyl)benzol, 5-Amino-2-di(2-hydroxyethyl)amino-(5-methyl-2-thienyl)benzol, 2,5-Diamino-(5-methyl-2-furyl)benzol, 2-Amino-5-di(2-hydroxyethyl)amino-(5-methyl-2-furyl)benzol, 5-Amino-2-di(2-hydroxyethyl)amino-(5-methyl-2-furyl)benzol, 2,5-Diamino-(3-ethyl-2-thienyl)benzol, 2-Amino-5-di(2-hydroxyethyl)amino-(3-ethyl-2-thienyl)benzol, 5-Amino-2-di(2-hydroxyethyl)amino-(3-ethyl-2-thienyl)benzol, 2,5-Diamino-(3-ethyl-2-furyl)benzol, 2-Amino-5-di(2-hydroxyethyl)amino-(3-ethyl-2-furyl)benzol, 5-Amino-2-di(2-hydroxyethyl)amino-(3-ethyl-2-furyl)benzol, 2,5-Diamino-(4-ethyl-2-thienyl)benzol, 2-Amino-5-di(2-hydroxyethyl)amino-(4-ethyl-2-thienyl)benzol, 5-Amino-2-di(2-hydroxyethyl)amino-(4-ethyl-2-thienyl)benzol, 2,5-Diamino-(4-ethyl-2-furyl)benzol, 2-Amino-5-di(2-hydroxyethyl)amino-(4-ethyl-2-furyl)benzol, 5-Amino-2-di(2-hydroxyethyl)amino-(4-ethyl-2-furyl)benzol, 2,5-Diamino-(5-ethyl-2-thienyl)benzol, 2-Amino-5-di(2-hydroxyethyl)amino-(5-ethyl-2-thienyl)benzol, 5-Amino-2-di(2-hydroxyethyl)amino-(5-ethyl-2-thienyl)benzol, 2,5-Diamino-(5-ethyl-2-furyl)benzol, 2-Amino-5-di(2-hydroxyethyl)-amino-(5-ethyl-2-furyl)benzol, 5-Amino-2-di(2-hydroxyethyl)amino-(5-ethyl-2-furyl)benzol, 2,5-Diamino-(3-dimethylamino-2-thienyl)benzol, 2-Amino-5-di(2-hydroxyethyl)amino-(3-dimethylamino-2-thienyl)benzol, 5-Amino-2-di(2-hydroxyethyl)amino-(3-dimethylamino-2-thienyl)benzol, 2,5-Diamino-(3-dimethylamino-2-furyl)benzol, 2-Amino-5-di(2-hydroxyethyl)amino-(3-dimethylamino-2-furyl)benzol, 5-Amino-2-di(2-hydroxyethyl)amino-(3-dimethylamino-2-furyl)benzol, 2,5-Diamino-(4-dimethylamino-2-thienyl)benzol, 2-Amino-5-di(2-hydroxyethyl)amino-(4-dimethylamino-2-thienyl)benzol, 5-Amino-2-di(2-hydroxyethyl)amino-(4-dimethylamino-2-thienyl)benzol, 2,5-Diamino-(4-dimethylamino-2-furyl)benzol, 2-Amino-5-di(2-hydroxyethyl)amino-(4-dimethylamino-2-furyl)benzol, 5-Amino-2-di(2-hydroxyethyl)amino-(4-dimethylamino-2-furyl)benzol, 2,5-Diamino-(5-dimethylamino-2-thienyl)benzol, 2-Amino-5-di(2-hydroxyethyl)amino-(5-dimethylamino-2-thienyl)benzol, 5-Amino-2-di(2-hydroxyethyl)amino-(5-dimethylamino-2-thienyl)benzol, 2,5-Diamino-(5-dimethylamino-2-furyl)benzol, 2-Amino-5-di(2-hydroxyethyl)amino-(5-dimethylamino-2-furyl)benzol, 5-Amino-2-di(2-hydroxyethyl)amino-(5-dimethylamino-2-furyl)benzol, 2,5-Diamino-(3-formyl-2-thienyl)benzol, 2-Amino-5-di(2-hydroxyethyl)amino-(3-formyl-2-thienyl)benzol, 5-Amino-2-di(2-hydroxyethyl)amino-(3-formyl-2-thienyl)benzol, 2,5-Diamino-(3-formyl-2-furyl)benzol, 2-Amino-5-di(2-hydroxyethyl)amino-(3-formyl-2-furyl)benzol, 5-Amino-2-di(2-hydroxyethyl)amino-(3-formyl-2-furyl)benzol, 2,5-Diamino-(4-formyl-2-thienyl)benzol, 2-Amino-5-di(2-hydroxyethyl)amino-(4-formyl-2-thienyl)benzol, 5-Amino-2-di(2-hydroxyethyl)amino(4-formyl-2-thienyl)benzol, 2,5-Diamino-(4-formyl-2-furyl)benzol, 2-Amino-5-di(2-hydroxyethyl)amino-(4-formyl-2-furyl)benzol, 5-Amino-2-di(2-hydroxyethyl)amino-(4-formyl-2-furyl)benzol, 2,5-Diamino(5-formyl-2-thienyl)benzol, 2-Amino-5-di(2-hydroxyethyl)amino-(5-formyl-2-thienyl)benzol, 5-Amino-2-di(2-hydroxyethyl)amino-(5-formyl-2-thienyl)benzol, 2,5-Diamino-(5-formyl-2-furyl)benzol, 2-Amino-5-di(2-hydröxyethyl)amino-(5-formyl-2-furyl)benzol, 5-Amino-2-di(2-hydroxyethyl)-amino(5-formyl-2-furyl)benzol, 2,5-Diamino-(3-acetyl-2-thienyl)benzol, 2-Amino-5-di(2-hydroxyethyl)amino-(3-acetyl-2-thienyl)benzol, 5-Amino-2-di(2-hydroxyethyl)amino-(3-acetyl-2-thienyl)benzol, 2,5-Diamino-(3-acetyl-2-furyl)benzol, 2-Amino-5-di(2-hydroxyethyl)amino-(3-acetyl-2-furyl)benzol, 5-Amino-2-di(2-hydroxyethyl)amino-(3-acetyl-2-furyl)benzol, 2,5-Diamino-(4-acetyl-2-thienyl)benzol, 2-Amino-5-di(2-hydroxyethyl)amino-(4-acetyl-2-thienyl)benzol, 5-Amino-2-di(2-hydroxyethyl)amino(4-acetyl-2-thienyl)benzol, 2,5-Diamino-(4-acetyl-2-furyl)benzol, 2-Amino-5-di(2-hydroxyethyl)amino-(4-acetyl-2-furyl)benzol, 5-Amino-2-di(2-hydroxyethyl)amino-(4-acetyl-2-furyl)benzol, 2,5-Diamino-(5-acetyl-2-thienyl)benzol, 2-Amino-5-di(2-hydroxyethyl)amino-(5-acetyl-2-thienyl)benzol, 5-Amino-2-di(2-hydroxyethyl)amino-(5-acetyl-2-thienyl)benzol, 2,5-Diamino-(5-acetyl-2-furyl)benzol, 2-Amino-5-di(2-hydroxyethyl)-amino(5-acetyl-2-furyl)benzol, 5-Amino-2-di(2-hydroxyethyl)amino(5-acetyl-2-furyl)benzol, 2,5-Diamino-(3-aminomethyl-2-thienyl)benzol, 2-Amino-5-di(2-hydroxyethyl)amino-(3-aminomethyl-2-thienyl)benzol, 5-Amino-2-di(2-hydroxyethyl)amino-(3-aminomethyl-2-thienyl)benzol, 2,5-Diamino-(3-aminomethyl-2-furyl)benzol, 2-Amino-5-di(2-hydroxyethyl)amino-(3-aminomethyl-2-furyl)benzol, 5-Amino-2-di(2-hydroxyethyl)amino-(3-aminomethyl-2-furyl)benzol, 2,5-Diamino-(4-aminomethyl-2-thienyl)benzol, 2-Amino-5-di(2-hydroxyethyl)amino-(4-aminomethyl-2-thienyl)benzol, 5-Amino-2-di(2-hydroxyethyl)amino-(4-aminomethyl-2-thienyl)benzol, 2,5-Diamino-(4-aminomethyl-2-furyl)benzol, 2-Amino-5-di(2-hydroxyethyl)amino-(4-aminomethyl-2-furyl)benzol, 5-Amino-2-di(2-hydroxyethyl)amino-(4-aminomethyl-2-furyl)benzol, 2,5-Diamino-(5-aminomethyl-2-thienyl)benzol, 2-Amino-5-di(2-hydroxyethyl)amino-(5-aminomethyl-2-thienyl)benzol, 5-Amino-2-di(2-hydroxyethyl)amino-(5-aminomethyl-2-thienyl)benzol, 2,5-Diamino-(5-aminomethyl-2-furyl)benzol, 2-Amino-5-di(2-hydroxyethyl)amino-(5-aminomethyl-2-furyl)benzol, 5-Amino-2-di(2-hydroxyethyl)amino-(5-aminomethyl-2-furyl)benzol, 2,5-Diamino-(3-(2-hydroxyethyl(imino))methylen-2-thienyl)benzol, 2-Amino-5-di(2-hydroxyethyl)amino-(3-(2-hydroxyethyl(imino))methylen-2-thienyl)benzol, 5-Amino-2-di(2-hydroxyethyl)amino-(3-(2-hydroxyethyl(imino))methylen-2-thienyl)benzol, 2,5-Diamino-(3-(2-hydroxyethyl(imino))methylen-2-furyl)benzol, 2-Amino-5-di(2-hydroxyethyl)amino-(3-(2-hydroxyethyl(imino))methylen-2-furyl)benzol, 5-Amino-2-di(2-hydroxyethyl)amino-(3-(2-hydroxyethyl(imino))methylen-2-furyl)benzol,2,5-Diamino-(4-(2-hydroxyethyl(imino))methylen-2-thienyl)benzol, 2-Amino-5-di(2-hydroxyethyl)amino-(4-(2-hydroxyethyl(imino))methylen-2-thienyl)benzol, 5-Amino-2-di(2-hydroxyethyl)amino-(4-(2-hydroxyethyl(imino))methylen-2-thienyl)benzol, 2,5-Diamino-(4-(2-hydroxyethyl(imino))methylen-2-furyl)benzol, 2-Amino-5-di(2-hydroxyethyl)amino-(4-(2-hydroxyethyl(imino))methylen-2-furyl)benzol, 5-Amino-2-di(2-hydroxyethyl)amino-(4-(2-hydroxyethyl(imino))methylen-2-furyl)benzol, 2,5-Diamino-(5-(2-hydroxyethyl(imino))methylen-2-thienyl)benzol, 2-Amino-5-di(2-hydroxyethyl)amino-(5-(2-hydroxyethyl(imino))methylen-2-thienyl)benzol, 5-Amino-2-di(2-hydroxyethyl)amino-(5-(2-hydroxyethyl(imino))methylen-2-thienyl)benzol, 2,5-Diamino-(5-(2-hydroxyethyl(imino))methylen-2-furyl)benzol, 2-Amino-5-di(2-hydroxyethyl)amino-(5-(2-hydroxyethyl(imino))methylen-2-furyl)benzol und 5-Amino-2-di(2-hydroxyethyl)amino-(5-(2-hydroxyethyl(imino))methylen-2-furyl)benzol.

Bevorzugt sind Verbindungen der Formel (I), bei denen (i) eine oder mehrere der Restgruppen R5, R6, R7 und R8 gleich Wasserstoff sind und/oder (ii) R1, R2, R3 und R4 gleichzeitig Wasserstoff bedeuten und/oder (iii) R7 gleich Wasserstoff und R6 gleich Wasserstoff, -C(O)H,-C(O)CH₃, C₁-C₄-Alkyl oder C₁-C₄-Hydroxyalkyl (insbesondere R6=R7=Wasserstoff) ist.

Insbesondere sind die folgenden Verbindungen zu nennen: 2,5-Diamino-1-(2-thienyl)benzol, 2,5-Diamino-1-(2-furyl)benzol, 2,5-Diamino-1-(3-acetyl-2-thienyl)benzol, 2,5-Diamino-1-(3-chlor-2-thienyl)benzol, 2,5-Diamino-1-(1-H-pyrrol-2-yl)benzol, 2,5-Diamino-1-(3-methyl-2-thienyl)benzol, 2,5-Diamino-1-(5-methyl-2-thienyl)benzol, 2,5-Diamino-1-(3-nitro-2-thienyl)benzol, 2-Dimethylamino-5-amino-1-(2-thienyl)-benzol, 2-Pyrrolidino-5-amino-1-(2-thienyl)-benzol, 2-Di(2-Hydroxyethyl)amino-5-amino-1-(2-thienyl)-benzol, 2-(2-Hydroxyethyl)amino-5-amino-1-(2-thienyl)-benzol, 2-(2-Methoxyethyl)amino-5-amino-1-(2-thienyl)-benzol, 2-(2,3-Dihydroxypropyl)amino-5-amino-1-(2-thienyl)-benzol, 2,5-Diamino-4-methoxy-1-(2-thienyl)-benzol, 2,5-Diamino-4-methyl-1-(2-thienyl)-benzol, 2,5-Diamino-1-(3-chloro-2-thienyl)benzol, 2,5-Diamino-1-(1-methyl-pyrrol-2-yl)benzol und 2,5-Diamino-1-(3-formyl-2-thienyl)benzol.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Das Diaminobenzol-Derivat der Formel (I) ist in dem erfindungsgemäßen Färbemittel in einer Menge von etwa 0,005 bis 20 Gewichtsprozent enthalten, wobei eine Menge von etwa 0,01 bis 5,0 Gewichtsprozent und insbesondere 0,1 bis 2,5 Gewichtsprozent bevorzugt ist.

Als Kupplersubstanzen kommen vorzugsweise 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxypyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diaminobenzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlorphenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxynaphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxybenzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxyindol,7-Hydroxy-indol und 2,3-Indolindion in Betracht.

Obwohl die vorteilhaften Eigenschaften der hier beschriebenen Diaminobenzol-Derivate der Formel (I) es nahelegen, diese als alleinige Entwicklersubstanz zu verwenden, ist es selbstverständlich auch möglich, die Diaminobenzol-Derivate der Formel (I) gemeinsam mit bekannten Entwicklersubstanzen, wie zum Beispiel 1,4-Diaminobenzol, 2,5-Diaminotoluol, 2,5-Diaminophenylethylalkohol, 4-Aminophenol und seinen Derivaten, beispielsweise 4-Amino-3-methylphenol, 4,5-Diaminopyrazol-Derivaten wie zum Beispiel 4,5-Diamino-1-(2-hydroxyethyl)-pyrazol oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6,0 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuß oder Unterschuß vorhanden sind.

Weiterhin kann das erfindungsgemäße Färbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'imino-2",5"-cyclohexadien-1"-yliden)-methyl]-2-methylaminobenzol-monohydrochlorid (C.I. 42 510) und 4-[(4'amino-3'-methyl-phenyl)-(4"-imino-3"-methyl-2",5"cyclohexadien-1"-yliden)-methyl]-2-methyl-aminobenzol monohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5-hydroxy-naphthalin-1-sulfonsäure-Natriumsalz (C.I. 14 805) und Dispersionsfarbstoffe wie beispielsweise 1,4-Diaminoanthrachinon und 1,4,5,8-Tetraaminoantrachinon, enthalten. Die Färbemittel können diese Farbkomponenten in einer Menge von etwa 0,1 bis 4,0 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein. Die Zubereitungsform des erfindungsgemäßen Färbemittels kann beispielsweise eine Lösung, insbesondere eine wäßrige oder wäßrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen pH-Wert von 6,8 bis 11,5 auf, wobei die basische Einstellung vorzugsweise mit Ammoniak erfolgt. Es können aber-auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Haarfärbemittel mit einem Gehalt an Diaminobenzol-Derivaten der Formel (I) als Entwicklersubstanz ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

Die in dem erfindungsgemäßen Mittel verwendeten Diaminobenzol-Derivate der Formel (I) sind gut in Wasser löslich und ermöglichen, Färbungen mit hoher Farbintensität und ausgezeichneter Farbechtheit, insbesondere was die Lichtechtheit, Waschechtheit und Reibeechtheit anbetrifft. Sie weisen weiterhin eine ausgezeichnete Lagerstabilität, insbesondere als Bestandteil der vorstehend beschriebenen Färbemittel, auf.

Ein weiterer Gegenstand der vorliegenden Erfindung sind neue Diaminobenzol-Derivat der Formel (I) 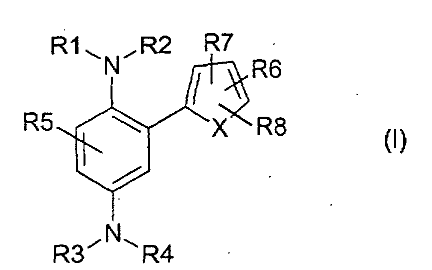 worin
**X** gleich Sauerstoff, Schwefel, Selen oder N-R9 ist;
**R1,R2,R3 und R4** unabhängig voneinander Wasserstoff, eine C₁-C₆-Alkylgruppe, eine C₁-C₄-Hydroxyalkylgruppe, eine C₂-C₄ Dihydroxyalkylgruppe oder eine C₁-C₄-Alkoxy-(C₁-C₂)-alkylgruppe darstellen oder R1und R2 beziehungsweise R3 und R4 einen viergliedrigen bis achtgliedrigen aliphatischen Ring bilden, wobei mindestens 2 der Reste R1 bis R4 Wasserstoff darstellen;
**R5** gleich Wasserstoff, einem Halogenatom, einer C₁-C₄-Alkylgruppe, einer, C₁-C₄-Hydroxyalkylgruppe oder einer C₁-C₄-Alkoxygruppe ist;
**R6 und R8** unabhänging voneinander gleich Wasserstoff, einer Hydroxygruppe, einem Halogenatom, einer Cyanogruppe, einer C₁-C₄-Alkoxygruppe, einer C₁-C₆ -Alkylgruppe, einer C₁-C₄-Alkylthioethergruppe, einer Mercaptogruppe, einer Nitrogruppe, einer Aminogruppe, einer Alkylaminogruppe, einer Dialkylaminogruppe, einer -C(O)H-Gruppe, einer -C(O)CH₃-Gruppe, einer -C(O)CF₃-Gruppe, einer -Si(CH₃)₃-Gruppe, einer C₁-C₄-Hydroxyalkylgruppe, einer C₃-C₄-Dihydroxyalkylgruppe, einer -CH=CHR10-Gruppe, einer -(CH₂)ₚ-CO₂R11-Gruppe oder einer -(CH₂)ₚ-R12-Gruppe mit p=1,2,3 oder 4, einer -C(R13)=NR14-Gruppe oder einer C(R16)H-NR17R18-Gruppe sind;
**R7** gleich Wasserstoff, einem Halogenatom, einer C₁-C₆ Alkylgruppe oder einer -C(O)H-Gruppe ist;
**R9** gleich Wasserstoff, einer C₁-C₆-Alkylgruppe, einer C₁-C₄-Hydroxyalkylgruppe, einer Phenylgruppe oder einer Acetylgruppe ist;
**R10** gleich Wasserstoff, einer Hydroxygruppe, einer Nitrogruppe, einer Aminogruppe, einer -CO₂R11-Gruppe oder einer -C(O)CH₃-Gruppe ist;
**R11, R13 und R16** unabhänging voneinander gleich Wasserstoff oder einer C₁-C₄-Alkylgruppe sind;
**R12** gleich einer Aminogruppe oder einer Nitrilgruppe ist;
**R14, R17 und R18** unabhängig voneinander gleich Wasserstoff, einer Hydroxygruppe, einer C₁-C₄-Alkylgruppe, einer C₁-C₄-Hydroxyalkylgruppe, einer C₃-C₄-Dihydroxyalkylgruppe oder einem Rest der Formel (II) sind 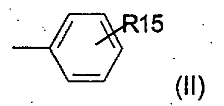 und
**R15** gleich Wasserstoff, einer Aminogruppe oder einer Hydroxygruppe ist, unter der Bedingung, daß, wenn gilt **X=** Sauerstoff und **R1**= **R2=** Methyl, mindestens einer der Reste **R3** bis **R8** verschieden von Wasserstoff ist, oder deren physiologisch verträgliche, wasserlösliche Salze.

Unter den neuen p-Diaminobenzol-Derivaten sind die Verbindungen der Formel (I), bei denen (i) eine oder mehrere der Restgruppen R5, R6, R7 und R8 gleich Wasserstoff sind und/oder (ii) R1, R2, R3 und R4 gleichzeitig Wasserstoff bedeuten und/oder (iii) R7 gleich Wasserstoff und R6 gleich Wasserstoff, -C(O)H, -C(O)CH₃, C₁-C₄-Alkyl oder C₁-C₄-Hydroxyalkyl (insbesondere R6=R7=Wasserstoff) ist, bevorzugt.

Insbesondere sind die folgenden Verbindungen zu nennen: 2,5-Diamino-1-(2-thienyl)benzol, 2,5-Diamino-1-(2-furyl)benzol, 2,5-Diamino-1-(3-acetyl-2-thienyl)benzol, 2,5-Diamino-1-(3-chlor-2-thienyl)benzol, 2,5-Diamino-1-(1-H-pyrrol-2-yl)benzol, 2,5-Diamino-1-(3-methyl-2-thienyl)benzol, 2,5-Diamino-1-(5-methyl-2-thienyl)benzol, 2,5-Diamino-1-(3-nitro-2-thienyl)benzol, 2-Dimethylamino-5-amino-1-(2-thienyl)-benzol, 2-Pyrrolidino-5-amino-1-(2-thienyl)-benzol, 2-Di(2-Hydroxyethyl)amino-5-amino-1-(2-thienyl)-benzol, 2-(2-Hydroxyethyl)amino-5-amino-1-(2-thienyl)-benzol, 2-(2-Methoxyethyl)amino-5-amino-1-(2-thienyl)-benzol, 2-(2,3-Dihydroxypropyl)amino-5-amino-1-(2-thienyl)-benzol, 2,5-Diamino-4-methoxy-1-(2-thienyl)-benzol, 2,5-Diamino-4-methyl-1-(2-thienyl)-benzol, 2,5-Diamino-1-(3-chloro-2-thienyl)benzol, 2,5-Diamino-1-(1-methyl-pyrrol-2-yl)benzol und 2,5-Diamino-1-(3-formyl-2-thienyl)benzol.

Die Herstellung der erfindungsgemäßen Diaminobenzol-Derivate der Formel (I) kann unter Verwendung von bekannten Syntheseverfahren, beispielsweise dem in den Ausführungsbeispielen beschriebenen Verfahren oder aber in Analogie zu dem in der EP-OS 0 604 353 für die Synthese des 4-N,N-Dimethylamino-3-(2-furyl)-anilin beschriebenen Verfahren, erfolgen.

Die nachfolgenden Beispiele sollen den Gegenstand der Erfindung näher erläutern, ohne ihn darauf zu beschränken.

### Beispiele

### Beispiele 1: Synthese von p-Diaminobenzolen der Formel (I) (Allgemeine Synthesevorschrift)

### A. Synthese von 2,5-tert.-Butyloxycarbonylamino-brombenzol

15,65g (0,07 mol) Brom-p-phenylendiamin-Hydrochlorid und 32,7 g (0,15 mol) Di-tert.-butyl-dicarbonat werden in einer Mischung von 250 ml 2N Natriunhydroxide und 250 ml Trifluortoluol gelöst und auf 45 °C erwärmt. Die Reaktionmischung wird 3 Tage gerührt. Schrittweise werden noch insgesamt 30 g (0,14 mol) Di-tert.-butyl-dicarbonat zugegeben. Anschließend wird die organische Schicht abgetrennt und die wäßrige Phase noch zweimal mit 100ml Dichlormethan extrahiert. Die vereinigten Extrakte weren eingedampft und der Rückstand in 200 ml Hexan aufgenommen. Der Niederschlag wird abfiltriert und mit 50 ml Hexan nachgewaschen.
Es werden 18,6 g (82 % der Theorie) 2,5-tert.-Butyloxycarbonylaminobrombenzol mit einem Schmelzpunkt von 130 °C erhalten.

### B. Synthese von 2,5-Diamino-1-(2-thienyl)-benzolen und 2,5-Diamino-1-(2-furyl)-benzolen

3,3 g (0,01 mol) 2,5-tert.-Butyloxycarbonylamino-brombenzol aus Stufe **A** und 0,013 mol der entsprechenden Borsäure werden unter Argon in 70 ml 1,2-Dimethoxyethan gelöst. Anschließend werden 0,5 g Tetrakis(triphenylphosphin)-palladium (0,0005 mol) und 13 ml 2N Kaliumcarbonat-lösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 40 ml Ethanol auf 50 °C erwärmt.
Anschließend werden zur Herstellung des Hydrochlorides 15 ml einer 2,9 molaren ethanolische Salzsäurelösung zugetropft. Der Niederschlag wird abfiltriert, zweimal mit 10 ml Ethanol gewaschen und sodann getrocknet.

### a. 2,5-Diamino-1-(2-thienyl)benzol-dihydrochlorid

Verwendete Borsäure: Thiophen-2-borsäure
Ausbeute: 1,6 g ( 61 % der Theorie)
Schmelzpunkt: 235 °Celsius (Zersetzung) (farblose Kristalle)

| CHN-Analyse: | | | |
|---|---|---|---|
| (C₁₀H₁₂N₂Cl₂S) | % C | % H | % N |
| berechnet | 45,64 | 4,60 | 10,64 |
| gefunden | 45,89 | 4,68 | 10,64 |

### b. 2,5-Diamino-1-(2-furyl)benzol-dihydrochlorid

Verwendete Borsäure: Furan-2-borsäure
Ausbeute: 1,6 g ( 63 % der Theorie)
Schmelzpunkt: 245 °Celsius (Zersetzung) (farblose Kristalle)

| CHN-Analyse: | | | |
|---|---|---|---|
| (C₁₀H₁₂N₂OCl₂) | % C | % H | % N |
| berechnet | 48,60 | 4,89 | 11,34 |
| gefunden | 48,78 | 4,48 | 11,31 |

### c. 2,5-Diamino-1-(3-acetyl-2-thienyl)benzol-dihydrochlorid

Verwendete Borsäure: (3-Acetyl-2-Thienyl)-borsäure
Ausbeute: 1,98g (65 % der Theorie)
Schmelzpunkt: 245 °Celsius (Zersetzung) (farblose Kristalle)

| CHN-Analyse: | | | |
|---|---|---|---|
| (C₁₂H₁₄N₂OCl₂S) | % C | % H | % N |
| berechnet | 47,22 | 4,62 | 9,18 |
| gefunden | 48,59 | 4,69 | 8,58 |

### d. 2.5-Diamino-1-(3-chlor-2-thienyl)benzol-dihydrochlorid

Verwendete Borsäure: (3-Chlor-2-Thienyl)-borsäure
Ausbeute: 1,7g ( 56 % der Theorie)
Schmelzpunkt: 245 °Celsius (Zersetzung) (farblose Kristalle)

| CHN-Analyse: | | | |
|---|---|---|---|
| (C₁₀H₉N₂ClS) | % C | % H | % N |
| berechnet | 40,45 | 3,73 | 9,41 |
| gefunden | 40,56 | 3,81 | 9,41 |

### e. 2,5-Diamino-1-(1-H-pyrrol-2-yl)benzol-dihydrochlorid

Verwendete Borsäure: 1-tert-Butoycarbonyl-pyrrol-2-borsäure
Ausbeute: 0.32g (13 % der Theorie)
Schmelzpunkt: 245 °Celsius (Zersetzung) (farblose Kristalle)

| CHN-Analyse: | | | |
|---|---|---|---|
| (C₁₀H₁₃N₃Cl₂) | % C | % H | % N |
| berechnet | 48.80 | 5.32 | 17.07 |
| gefunden | 48.68 | 4.98 | 16.47 |

### C. Synthese von N,N'-Bis(tert-Butoxycarbonyl)-2,5-diamino-1-phenylborsäure

Die N,N'-Bis(tert-Butoxycarbonyl)-2,5-diamino-1-phenylborsäure werden durch Umsetzung von N,N'-Bis(tert-Butoxycarbonyl)-2,5-diamino-1-brombenzol mit tert-Butyllithium und Trimethylborate dargestellt. Die experimentelle Vorschrift dieser Herstellungsmethode wird von J. M. Tour und J. J. S: Lamba in J. Am. Chem. Soc.1994,116 Seite 11723 beschrieben.

### D. Synthese von 2,5-Diamino-1-(2-thienyl)-benzolen

0,035 g (0,0001 mol) 2,5-tert.-Butyloxycarbonylamino-1-phenylborsäure aus Stufe **C** und 0,00015 mol des entsprechenden Bromderivates werden unter Argon in 10 ml 1,2-Dimethoxyethan gelöst. Anschließend werden 0,005 g Tetrakis-(triphenylphosphin)-palladium (0,000005 mol) und 0,13 ml 2N Kaliumcarbonat-lösung zugegeben und die Reaktionsmischung auf 80 °C erwärmt. Nach Beendigung der Reaktion wird die Reaktionsmischung in 10 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt.
Anschließend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.

### a. 2,5-Diamino-1-(3-methyl-2-thienyl)benzol-dihydrochlorid

Verwendete Bromderivat: 2-Brom-5-methylthiophen
Ausbeute: 0.025 g ( 71 % der Theorie)
Schmelzpunkt: 245 °Celsius (Zersetzung) (farblose Kristalle)

### b. 2,5-Diamino-1-(5-methyl-2-thienyl)benzol-dihydrochlorid

Verwendete Bromderivat: 2-Brom-3-methylthiophen
Ausbeute: 0.025 g ( 80 % der Theorie)
Schmelzpunkt: 245 °Celsius (Zersetzung) (farblose Kristalle)

### c. 2,5-Diamino-1-(3-nitro-2-thienyl)benzol-dihydrochlorid

Verwendete Bromderivat: 2-Brom-5-nitrothiophen
Ausbeute: 0.025 g ( 71 % der Theorie)
Masspektren MH⁺ 205 (100)

### Beispiel 2: Synthese von 2-N-substituierten-Amino-5-amino-1-(2-thienyl)-benzolen (Allgemeine Synthesevorschrift)

### A. Synthese von 2-Fluor-5-nitro-1-(2-thienyl)-benzol

1,75 g (0,01 mol) 1-Chlor-2-fluoro-5-nitrobenzol und 0,013 mol Thiophen-2-borsäure werden unter Argon in 70 ml 1,2-Dimethoxyethan gelöst. Anschließend werden 0,5 g Tetrakis-(triphenylphosphin)-palladium (0,0005 mol) und 13 ml 2N Kaliumcarbonat-lösung zugegeben und die Reaktionsmischung auf 80 °C erwärmt. Nach Beendigung der Reaktion wird die Reaktionsmischung in 100 ml Essigsäureethylestergegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Hexan/Essigsäureethylester (20:1) gereinigt.
Es werden 1,24 g (56 % der Theorie) 2-Fluor-5-nitro-1-(3-thienyl)-benzol mit einem Schmelzpunkt von 65 °C erhalten.

### B. Synthese von 2-N-substituierten 2-Amino-5-amino-1-(2-thienyl)benzolen

0,56 g (0,0025 mol) 2-Fluor-5-nitro-1-(3-thienyl)-benzol aus Stufe **A** und 5 ml des entsprechenden Amins werden in 5 ml Ethanol gelöst. Anschließend wird die Reaktionmischung auf 80 °C erwärmt. Nach Beendigung der Reaktion wird die Reaktionmischung in 50 g Eis gegossen, mit Essigsäureethylester extrahiert und sodann mit Magnesiumsulfat getrocknet. Das Lösungsmittel wird am Rotationsverdampfer abdestilliert und der Rückstand an Kieselgel mit Dichlormethan/Ethanol (50:1) gereinigt. Das so erhaltene Produkt wird in 30 ml Ethanol gelöst und unter Zusatz von 100 mg eines Palladium-Aktivkohle-Katalysators (10 %ig) bei 50 °Celsius hydriert. Nach Aufnahme der erforderlichen Wasserstoff-menge wird vom Katalysator abfiltriert und das Lösungsmittel am Rotationsverdampfer abdestilliert. Anschließend werden zur Herstellung des Hydrochlorides 5 ml einer 2,9 molaren ethanolischen Salzsäurelösung zugetropft. Der Niederschlag wird abfiltriert, zweimal mit 10 ml Ethanol gewaschen und sodann getrocknet.

### a. 2-Dimethylamino-5-amino-1-(2-thienyl)-benzol-dihydrochlorid

Verwendetes Amin: Dimethylamin
Ausbeute: 0,58 g (77 % der Theorie)
Schmelzpunkt: 232 °Celsius (Zersetzung) (farblose Kristalle)

| CHN-Analyse: | | | |
|---|---|---|---|
| (C₁₂H₁₆N₂Cl₂S) | % C | % H | % N |
| berechnet | 49,48 | 5,54 | 9,61 |
| gefunden | 48,65 | 5,48 | 9,39 |

### b. 2-Pyrrolidino-5-amino-1-(2-thienyl)-benzol-dihydrochlorid

Verwendetes Amin: Pyrrolidin
Ausbeute: 0,69 g (69 % der Theorie)
Schmelzpunkt: 205 °Celsius (Zersetzung) (farblose Kristalle)

| CHN-Analyse: | | | |
|---|---|---|---|
| (C₁₄H₁₈N₂S) | % C | % H | % N |
| berechnet | 52,99 | 5,72 | 8,83 |
| gefunden | 52,58 | 5,93 | 8,59 |

### c. 2-Di(2-Hydroxyethyl)amino-5-amino-1-(2-thienyl)-benzol-dihydrochlorid

Verwendetes Amin: Diethanolamin
Ausbeute: 0,76 g (87 % der Theorie)
Schmelzpunkt: 208 °Celsius (Zersetzung) (farblose Kristalle)

| CHN-Analyse: | | | |
|---|---|---|---|
| (C₁₄H₂₀N₂O₂Cl₂S) | % C | % H | % N |
| berechnet | 47,86 | 5,74 | 7,97 |
| gefunden | 47,65 | 5,67 | 7,83 |

### d. 2-(2-Hydroxyethyl)amino-5-amino-1-(2-thienyl)-benzol-dihydrochlorid

Verwendetes Amin: Ethanolamin
Ausbeute: 0,95 g (87 % der Theorie)
Schmelzpunkt: 208 °Celsius (Zersetzung) (farblose Kristalle)

| CHN-Analyse: | | | |
|---|---|---|---|
| (C₁₂H₁₆N₂OCl₂S) | % C | % H | % N |
| berechnet | 46,91 | 5,25 | 9,12 |
| gefunden | 47,1 | 5,32 | 9,16 |

### e. 2-(2-Methoxyethyl)amino-5-amino-1-(2-thienyl)-benzol-dihydrochlorid

Verwendetes Amin 2-Methoxyethylamin
Ausbeute: 0,60 g (79 % der Theorie)
Schmelzpunkt: 208 °Celsius (Zersetzung) (farblose Kristalle)

| CHN-Analyse: | | | |
|---|---|---|---|
| (C₁₃H₁₈Cl₂N₂OS) | % C | % H | % N |
| berechnet | 48,60 | 5,65 | 8,72 |
| gefunden | 51,00 | 6,37 | 8,08 |

### f. 2-(2,3-Dihydroxypropyl)amino-5-amino-1-(2-thienyl)-benzoldihydrochlorid

Verwendetes Amin 2,3-Dihydroxypropylamin
Ausbeute: 0,71 g (85 % der Theorie)
Schmelzpunkt: 208 °Celsius (Zersetzung) (farblose Kristalle)

| CHN-Analyse: | | | |
|---|---|---|---|
| (C₁₃H₁₈Cl₂N₂O₂S) | % C | % H | %N |
| berechnet | 46,3 | 5,38 | 8,31 |
| gefunden | 48,80 | 6,21 | 8,20 |

### Beispiel 3: Synthese von 2,5-Diamino-4-methyl-1-(2-thienyl)-benzol

1,87 g (0,01 mol) 5-Chlor-2-methyl-4-nitroanilin und 0,013 mol Thiophen-2-borsäure werden unter Argon in 70 ml 1,2-Dimethoxyethan gelöst. Anschließend werden 0,5 g Tetrakis-(triphenylphosphin)-palladium (0,0005 mol) und 13 ml 2N Kaliumcarbonat-lösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Hexan/Essigsäureethylester (20:1) gereinigt.
Das so erhaltene Produkt wird in 30 ml Ethanol gelöst und unter Zusatz von 100 mg eines Palladium-Aktivkohle-Katalysators (10 %ig) bei 50 °Celsius hydriert. Nach Aufnahme der erforderlichen Wasserstoff-menge wird vom Katalysator abfiltriert und das Lösungsmittel am Rotationsverdampfer abdestilliert.
Anschließend werden zur Herstellung des Hydrochlorides 5 ml einer 2,9 molaren ethanolischen Salzsäurelösung zugetropft. Der Niederschlag wird abfiltriert, zweimal mit 10 ml Ethanol gewaschen und sodann getrocknet.
Ausbeute: 0,2 g (10 % der Theorie)

| CHN-Analyse: | | | |
|---|---|---|---|
| (C₁₁H₁₄Cl₂N₂S) | % C | % H | % N |
| berechnet | 47,66 | 5,09 | 10,11 |
| gefunden | 44,64 | 5,58 | 10,57 |

### Beispiel 4: Synthese von 2,5-Diamino-4-methoxy-1-(2-thienyl)-benzol

1,87 g (0,01 mol) 5-Chlor-2-methoxy-4-nitroanilin und 0,013 mol Thiophen-2-borsäure werden unter Argon in 70 ml 1,2-Dimethoxyethan gelöst. Anschließend werden 0,5 g Tetrakis-(triphenylphosphin)-palladium (0,0005 mol) und 13 ml 2N Kaliumcarbonat-lösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Hexan/Essigsäureethylester (20:1) gereinigt.
Das so erhaltene Produkt wird in 30 ml Ethanol gelöst und unter Zusatz von 100 mg eines Palladium-Aktivkohle-Katalysators (10 %ig) bei 50 °Celsius hydriert. Nach Aufnahme der erforderlichen Wasserstoff-menge wird vom Katalysator abfiltriert und das Lösungsmittel am Rotationsverdampfer abdestilliert.
Anschließend werden zur Herstellung des Hydrochlorides 5 ml einer 2,9 molaren ethanolischen Salzsäurelösung zugetropft. Der Niederschlag wird abfiltriert, zweimal mit 10 ml Ethanol gewaschen und sodann getrocknet.
Ausbeute: 0,53 g (30 % der Theorie)

| CHN-Analyse: | | | |
|---|---|---|---|
| (C₁₁H₁₄Cl₂N₂OS) | % C | % H | % N |
| berechnet | 45,06 | 4,81 | 9,55 |
| gefunden | 42,92 | 5,29 | 9,26 |

### Beispiele 5 bis 71: Haarfärbemittel

Es werden Haarfärbelösungen der folgenden Zusammensetzunq hergestellt:

| | |
|---|---|
| 0,0125 mol | Entwicklersubstanz der Formel (I) gemäß Tabelle 1 |
| 0,0125 mol | Kupplersubstanz gemäß Tabelle 1 |
| 10,0 g | Kaliumoleat (8prozentige wäßrige Lösung) |
| 10,0 g | Ammoniak (22prozentige wäßrige Lösung) |
| 10,0 g | Isopropanol |
| 0,3 g | Ascorbinsäure |
| ad 100,0g | Wasser |

30 g der vorstehenden Färbelösung werden unmittelbar vor der : Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die resultierenden Färbungen sind in Tabelle 1 zusammengefaßt.

**Tabelle 1:**

| **Beispiel** | **Entwicklersubstanz der Formel (I)** | **Kupplersubstanz** | **erhaltene Färbung** |
|---|---|---|---|
| **5** | 2,5-Diamino-1-(2- thienyl)benzol∗2HCl | 2-Amino-4-(2'-hydroxyethyl)-amino-anisolsulfat | dunkelblau |
| **6** | 2,5-Diamino-1-(2-thienyl)benzol∗2HCl | m-Aminophenol | dunkelgrau |
| **7** | 2,5-Diamino-1-(2-thienyl)benzol∗2HCl | 5-Amino-2-methylphenol | rot |
| **8** | 2,5-Diamino-1-(2-thienyl)benzol∗2HCl | Resorcin | dunkelblond |
| **9** | 2,5-Diamino-1-(2-thienyl)benzol∗2HCl | 1-Chlor-2,4-dihydroxybenzol | dunkelblond |
| **10** | 2,5-Diamino-1-(2-thienyl)benzol∗2HCl | 1,3-Diaminobenzol | dunkelblau |
| **11** | 2,5-Diamino-1-(2-thienyl)benzol∗2HCl | 1-Naphthol | dunkelrotblau |
| **12** | 2,5-Diamino-1-(2-thienyl)benzol∗2HCl | 5-Hydroxy-1,3-benzodioxol | dunkelblond |
| **13** | 2,5-Diamino-1-(2-thienyl)benzol∗2HCl | 3-Amino-2-chlor-6-methyl-phenol | dunkelrotblau |
| **14** | 2,5-Diamino-1-(2-thienyl)benzol∗2HCl | 3-Amino-6-methoxy-2-(methylamino)pyridin∗ 2HCl | dunkelbalu |
| **15** | 2,5-Diamino-1-(2-thienyl)benzol∗2HCl | 1,3-Di-(2,4-diaminophenoxy)propan*4H | dunkelblau |
| **16** | 2,5-Diamino-1-(2-thienyl)benzol∗2HCl | 1,3-Diamino-4-(2-hydroxyethoxy)benzol | dunkelblau |
| **17** | 2,5-Diamino-1-(2-thienyl)benzol∗2HCl | 1,3-Dihydroxy-2-methyl-benzol | blond |
| **18** | 2,5-Diamino-1-(2-thienyl)benzol∗2HCl | 5-((2-hydroxyethyl)amino)2-methylphenol | rot |
| **19** | 2,5-Diamino-1-(2-thienyl)benzol∗2HCl | 1,5-Dihydroxy-naphthalin | blau |
| **20** | 2,5-Diamino-1-(2-thienyl)benzol∗2HCl | 1,7-Dihydroxynaphthalin | rot blau |
| **21** | 2,5-Diamino-1-(2-thienyl)benzol∗2HCl | 5-((2-Hydroxyethyl)amino)-1,3-bezodioxol∗HCl | dunkelblond |
| **22** | 2,5-Diamino-1-(2-thienyl)benzol∗2HCl | Essigsäure-(2-methyl-naphthalin-1-yl)-ester | violet |
| **23** | 2,5-Diamino-1-(2-thienyl)benzol∗2HCl | 5,6-Dihydroxy-1H-indol | blond |
| **24** | 2,5-Diamino-1-(2-furyl)benzol∗2HCl | 2-Amino-4-(2'-hydroxyethyl)-amino-anisolsulfat | dunkelblau |
| **25** | 2,5-Diamino-1-(2-furyl)benzol∗2HCl | m-Aminophenol | dunkelgrau |
| **26** | 2,5-Diamino-1-(2-furyl)benzol∗2HCl | 5-Amino-2-methylphenol | rot |
| **27** | 2,5-Diamino-1-(2-furyl)benzol∗2HCl | Resorcin | dunkelblond |
| **28** | 2,5-Diamino-1-(3-acetyl-2-thienyl)benzol∗2HCl | 2-Amino-4-(2'-hydroxyethyl)-amino-anisolsulfat | dunkelblau |
| **29** | 2,5-Diamino-1-(3-acetyl-2-thienyl)benzol∗2HCl | m-Aminophenol | dunkelgrau |
| **30** | 2,5-Diamino-1-(3-acetyl-2-thienyl)benzol∗2HCl | 5-Amino-2-methylphenol | rot |
| **31** | 2,5-Diamino-1-(3-acetyl-2-thienyl)benzol∗2HCl | Resorcin | dunkelblond |
| **32** | 2,5-Diamino-1-(3-chlor-2-thienyl)benzol∗2HCl | 2-Amino-4-(2'-hydroxyethyl)-amino-anisolsulfat | dunkelblau |
| **33** | 2,5-Diamino-1-(3-chlor-2-thienyl)benzol∗2HCl | m-Aminophenol | dunkelgrau |
| **34** | 2,5-Diamino-1-(3-chlor-2-thienyl)benzol∗2HCl | 5-Amino-2-methylphenol | rot |
| **35** | 2,5-Diamino-1-(3-chlor-2-thienyl)benzol∗2HCl | Resorcin | dunkelblond |
| **36** | 2-Di(2-Hydroxyethyl)amino-5-amino-1-(2-, thienyl)benzol∗2HCl | 2-Amino-4-(2-hydroxyethyl)-amino-anisolsulfat | blaurot |
| **37** | 2-Di(2-Hydroxyethyl)amino-5-amino-1-(2-thienyl)benzol∗2HCl | 3-Aminophenol | grau |
| **38** | 2-Di(2-Hydroxyethyl)amino-5-amino-1-(2-thienyl)benzol∗2HCl | 5-Amino-2-methylphenol | rot |
| **39** | 2-Di(2-Hydroxyethyl)amino-5-amino-1-(2-thienyl)benzol∗2HCl | 1,3-Dihydroxybenzol | blond |
| **40** | 2-Pyrrolidino-5-amino-1-(2-thienyl)benzol∗2HCl | 2-Amino-4-(2-hydroxyethyl)-amino-anisolsulfat | blau |
| **41** | 2-Pyrrolidino-5-amino-1-(2-thienyl)benzol∗2HCl | 3-Aminophenol | grau |
| **42** | 2-Pyrrolidino-5-amino-1-(2-thienyl)benzol∗2HCl | 5-Amino-2-methylphenol | rot |
| **43** | 1,3-Dihydroxybenz2-Amino-4-(2-hydroxyethyl)amino-anisolsulfat | 1,3-Dihydroxybenzol | blond |
| **44** | 2-Dimethylamino-5-amino-1-(2-thienyl)benzol∗2HCl | 2-Amino-4-(2-hydroxyethyl)amino-anisolsulfat | blau |
| **45** | 2-Dimethylamino-5-amino-1-(2-thienyl)benzol∗2HCl | 3-Aminophenol | grau |
| **46** | 2-Dimethylamino-5-amino-1-(2-thienyl)benzol∗2HCl | 5-Amino-2-methylphenol | rot |
| **47** | 2-Dimethylamino-5-amino-1-(2-thienyl)benzol∗2HCl | 1,3-Dihydroxybenzol | blond |
| **48** | 2-(2-Hydroxyethyl)amino-5-amino-1-(2-thienyl)benzol∗2HCl | 2-Amino-4-(2-hydroxyethyl)amino-anisolsulfat | blau |
| **49** | 2-(2-Hydroxyethyl)amino-5-amino-1-(2-thienyl)benzol∗2HCl | 3-Aminophenol | grau |
| **50** | 2-(2-Hydroxyethyl)amino-5-amino-1-(2-thienyl)benzol∗2HCl | 5-Amino-2-methylphenol | rot |
| **51** | 2-(2-Hydroxyethyl)amino-5-amino-1-(2-thienyl)benzol∗2HCl | 1,3-Dihydroxybenzol | blond |
| **52** | 2-(2-Methoxyethyl)amino-5-amino-1-(2-thienyl)benzol∗2HCl | 2-Amino-4-(2-hydroxyethyl)amino-anisolsulfat | blau |
| **53** | 2-(2-Methoxyethyl)amino-5-amino-1 -(2-thienyl)benzol∗2HCl | 3-Aminophenol | grau |
| **54** | 2-(2-Methoxyethyl)amino-5-amino-1-(2-thienyl)benzol∗2HCl | 5-Amino-2-methylphenol | rot |
| **55** | 2-(2-Methoxyethyl)amino-5-amino-1-(2-thienyl)benzol∗2HCl | 1,3-Dihydroxybenzol | blond |
| **56** | 2-(2,3-Dihydroxypropyl)amino-5-amino-1-(2-thienyl)-benzol∗2HCl | 2-Amino-4-(2-hydroxyethyl)amino-anisolsulfat | blau |
| **57** | 2-(2,3-Dihydroxypropyl)amino-5-amino-1-(2-thienyl)-benzol∗2HCl | 3-Aminophenol | grau |
| **58** | 2-(2,3-Dihydroxypropyl)amino-5-amino-1-(2-thienyl)-benzol∗2HCl | 5-Amino-2-methylphenol | rot |
| **59** | 2-(2,3-Dihydroxypropyl)amino-5-amino-1-(2-thienyl)-benzol∗2HCl | 1,3-Dihydroxybenzol | blond |
| **60** | 2,5-Diamino-1-(1-H-pyrrol-2-yl)benzol-dihydrochlorid | 2-Amino-4-(2-hydroxyethyl)amino-anisolsulfat | blau |
| **61** | 2,5-Diamino-1-(1-H-pyrrol-2-yl)benzol-dihydrochlorid | 3-Aminophenol | grau |
| **62** | 2,5-Diamino-1-(1-H-pyrrol-2-yl)benzol-dihydrochlorid | 5-Amino-2-methylphenol | rot |
| **63** | 2,5-Diamino-1-(1-H-pyrrol-2-yl)benzol-dihydrochlorid | 1,3-Dihydroxybenzol | blond |
| **64** | 2,5-Diamino-4-methoxy-1-(2-thienyl)-benzol | 2-Amino-4-(2-hydroxyethyl)amino-anisolsulfat | blau |
| **65** | 2,5-Diamino-4-methoxy-1-(2-thienyl)-benzoldihydrochlorid | 3-Aminophenol | blau-grün |
| **66** | 2,5-Diamino-4-methoxy-1-(2-thienyl)-benzoldihydrochlorid | 5-Amino-2-methylphenol | violett |
| **67** | 2,5-Diamino-4-methoxy-1-(2-thienyl)-benzoldihydrochlorid | 1,3-Dihydroxybenzol | violett |
| **68** | 2,5-Diamino-4-methyl-1-(2-thienyl)-benzoldihydrochlorid | 2-Amino-4-(2-hydroxyethyl)amino-anisolsulfat | blau |
| **69** | 2,5-Diamino-4-methyl-1-(2-thienyl)-benzoldihydrochlorid | 3-Aminophenol | grau |
| **70** | 2,5-Diamino-4-methyl-1-(2-thienyl)-benzoldihydrochlorid | 5-Amino-2-methylphenol | rot |
| **71** | 2,5-Diamino-4-methyl-1-(2-thienyl)-benzoldihydrochlorid | 1,3-Dihydroxybenzol | blond |

### Beispiele 72 bis 83: Haarfärbemittel

Es werden Haarfärbelösungen der folgenden Zusammensetzung hergestellt:

| | |
|---|---|
| 0,0000125 mol | Entwicklersubstanz der Formel (I) gemäß Tabelle 2 |
| 0,0000125 mol | Kupplersubstanz gemäß Tabelle 2 |
| 0,01g | Kaliumoleat (8prozentige wäßrige Lösung) |
| 0,01g | Ammoniak (22prozentige wäßrige Lösung) |
| 0,01g | Ethanol |
| 0,003 g | Ascorbinsäure |
| ad 1,0 g | Wasser |

1 g der vorstehenden Färbelösung werden unmittelbar vor der Anwendung mit 1 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die resultierenden Färbungen sind in Tabelle 2 zusammengefaßt.

**Tabelle 2**

| **Beispiel** | **Entwicklersubstanz der Formel (I)** | **Kupplersubstanz** | **erhaltene Färbung** |
|---|---|---|---|
| **72** | 2,5-Diamino-1-(3-methyl-2-thienyl)benzol∗2HCl | 1,3-Diamino-4-(2-hydroxyethoxy)benzol | dunkelblau |
| **73** | 2,5-Diamino-1-(3-methyl-2-thienyl)benzol∗2HCl | 1-Naphtol | blau |
| **74** | 2,5-Diamino-1-(3-methyl-2-thienyl)benzol∗2HCl | 5-Amino-2-methyl-phenol | rot |
| **75** | 2,5-Diamino-1-(3-methyl-2-thienyl)benzol∗2HCl | Resorcin | dunkelblond |
| **76** | 2,5-Diamino-1-(5-methyl-2-thienyl)benzol∗2HCl | 1,3-Diamino-4-(2-hydroxyethoxy)benzol | dunkelblau |
| **77** | 2,5-Diamino-1-(5-methyl-2-thienyl)benzol∗2HCl | 1-Naphtol | blau |
| **78** | 2,5-Diamino-1-(5-methyl-2-thienyl)benzol∗2HCl | 5-Amino-2-methyl-phenol | rot |
| **79** | 2,5-Diamino-1-(5-methyl-2-thienyl)benzol∗2HCl | Resorcin | dunkelblond |
| **80** | 2,5-Diamino-1-(3-nitro-2-thienyl)benzol∗2HCl | 1,3-Diamino-4-(2-hydroxyethoxy)benzol | dunkelblau |
| **81** | 2,5-Diamino-1-(3-nitro-2-thienyl)benzol∗2HCl | 1-Naphtol | blau |
| **82** | 2,5-Diamino-1-(3-nitro-2-thienyl)benzol∗2HCl | 5-Amino-2-methyl-phenol | rot |
| **83** | 2,5-Diamino-1-(3-nitro-2-thienyl)benzol∗2HCl | Resorcin | dunkelblond |

### Beispiele 84 bis 124: Haarfärbemittel

Es werden Haarfärbelösungen der folgenden Zusammensetzung hergestellt:

| | |
|---|---|
| 0,000625 mol | Entwicklersubstanz der Formel (I) gemäß Tabelle 3 |
| 0,000625 mol | Entwicklersubstanz gemäß Tabelle 3 |
| 0,001250 mol | Kupplersubstanz gemäß Tabelle 3 |
| 10,0 g | Kaliumoleat (8prozentige wäßrige Lösung) |
| 10,0 g | Ammoniak (22prozentige wäßrige Lösung) |
| 10,0 g | Isopropanol |
| 0,3 g | Ascorbinsäure |
| ad 100,0 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die resultierenden Färbungen sind in Tabelle 2 zusammengefaßt.

**Tabelle 3:**

| **Beispiel** | **Entwicklersubstanz der Formel (I)/ Entwicklersubstanz** | **Kupplersubstanz** | **erhaltene Färbung** |
|---|---|---|---|
| **84** | 2,5-Diamino-1-(2-thienyl)benzol∗2HCl/1,4-Diamino-benzol | 2-Amino-4-(2'-hydroxyethyl)amino-anisolsulfat | dunkelblau |
| **85** | 2,5-Diamino-1-(2-thienyl)benzol∗2HCl /1,4-Diamino-benzol | 1 ,3-Dihydroxy-2-methyl-benzol | blond |
| **86** | 2,5-Diamino-1-(2-thienyl)benzol∗2HCl /1,4-Diamino-benzol | 5-((2-Hydroxyethyl)amino)-1,3-benzodioxol-hydrochlorid | dunkelblond - olivgrün |
| **87** | 2,5-Diamino-1-(2-thienyl)benzol∗2HCl /1,4-Diaminobenzol | 5-Amino-2-methylphenol | rotblau |
| **88** | 2,5-Diamino-1-(2-thienyl)benzol∗2HCl /1,4-Diamino-benzol | 3-Amino-phenol | grau |
| **89** | 2,5-Diamino-1-(2-thienyl)benzol∗2HCl/1,4-Diamino-benzol | 1,3-Diamino-4-(2-hydroxyethoxy)benzol | dunkelblau |
| **90** | 2,5-Diamino-1-(2-thienyl)benzol∗2HCl/1,4-Diaminobenzol | 1,3-Dihydroxybenzol | blond |
| **91** | 2,5-Diamino-1-(2-thienyl)benzol∗2HCl/1,4-Diamino-2-methyl-benzol | 2-Amino-4-(2'-hydroxyethyl)amino-anisolsulfat | dunkelblau |
| **92** | 2,5-Diamino-1-(2-thienyl)benzol∗2HCl/1,4-Diamino-2-methyl-benzol | 1,3-Dihydroxy-2-methyl-benzol | blond |
| **93** | 2,5-Diamino-1-(2-thienyl)benzol∗2HCl/1,4-Diamino-2-methyl-benzol | 5-((2-Hydroxyethyl)amino)-1,3-benzodioxol∗HCl | dunkelblond - olivgrün |
| **94** | 2,5-Diamino-1-(2-thienyl)benzol∗2HCl/1,4-Diamino-2-methyl-benzol | 5-Amino-2-methylphenol | violett |
| **95** | 2,5-Diamino-1-(2-thienyl)benzol∗2HCl/1,4-Diamino-2-methyl-benzol | 3-Amino-phenol | grau-rot |
| **96** | 2,5-Diamino-1-(2-thienyl)benzol∗2HCl/1,4-Diamino-2-methyl-benzol | 1,3-Diamino-4-(2-hydroxyethoxy)benzol | dunkelblau |
| **97** | 2,5-Diamino-1-(2-thienyl)benzol∗2HCl /1,4-Diamino-2-methyl-benzol | 1,3-Dihydroxybenzol | blond |
| **98** | 2,5-Diamino-1-(2-thienyl)benzol∗2HCl/4-Di(2-hydroxyethyl)amino-anilinsulfat | 2-Amino-4-(2'-hydroxyethyl)amino-anisolsulfat | dunkelblau |
| **99** | 2,5-Diamino-1-(2-thienyl)benzol∗2HCl/4-Di-(2-hydroxyethyl)amino-anilinsulfat | 1,3-Dihydroxy-2-methyl-benzol | braun |
| **100** | 2,5-Diamino-1-(2-thienyl)benzol∗2HCl/4-Di-(2-hydroxyethyl)amino-anilinsulfat | 5-((2-Hydroxyethyl)amino)-1,3-benzodioxol*HCl | dunkelblond - olivgrün |
| **101** | 2,5-Diamino-1-(2-thienyl)benzol∗2HCl/4-Di-(2-hydroxyethyl)amino-anilinsulfat | 5-Amino-2-methylphenol | violett |
| **102** | 2,5-Diamino-1-(2-thienyl)benzol∗2HCl/4-Di-(2-hydroxyethyl)amino-anilinsulfat | 3-Amino-phenol | grau-blau |
| **103** | 2,5-Diamino-1-(2-thienyl)benzol∗2HCl/4-Di-(2-hydroxyethyl)amino-anilin-sulfat | 1,3-Diamino-4-(2-hydroxyethoxy)benzol | dunkelblau |
| **104** | 2,5-Diamino-1-(2-thienyl)benzol∗2HCl/4-Di-(2-hydroxyethyl)amino-anilin-sulfat | 1,3-Dihydroxybenzol | blond |
| **105** | 2,5-Diamino-1-(2-thienyl)benzol∗2HCl /4-Amino-phenol | 2-Amino-4-(2'-hydroxyethyl)amino-anisolsulfat | violett |
| **106** | 2,5-Diamino-1-(2-thienyl)benzol∗2HCl/4-Amino-phenol | 1,3-Dihydroxy-2-methyl-benzol | blond |
| **107** | 2,5-Diamino-1-(2-thienyl)benzol∗2HCl/4-Amino-phenol | 5-((2-Hydroxyethyl)amino)-1,3-benzodioxol∗HCl | blond - olivgrün |
| **108** | 2,5-Diamino-1-(2-thienyl)benzol∗2HCl/4-Aminophenol | 5-Amino-2-methylphenol | rot |
| **109** | 2,5-Diamino-1-(2-thienyl)benzol∗2HCl/4-Amino-phenol | 3-Amino-phenol | braun |
| **110** | 2,5-Diamino-1-(2-thienyl)benzol∗2HCl/4-Amino-phenol | 1,3-Diamino-4-(2-hydroxyethoxy)benzol | dunkelblau |
| **111** | 2,5-Diamino-1-(2-thienyl)benzol*2HCl / 4-Amino-phenol | 1,3-Dihydroxybenzol | blond |
| **112** | 2,5-Diamino-1-(2-thienyl)benzol∗2HCl /2,4,5,6-Tetraamino-pyrimindin-sulfat | 2-Amino-4-(2'-hydroxyethyl)amino-anisolsulfat | dunkelblau |
| **113** | 2,5-Diamino-1-(2-thienyl)benzol∗2HCl /2,4,5,6-Tetraamino-pyrimindin-sulfat | 1,3-Dihydroxy-2-methyl-benzol | rot |
| **114** | 2,5-Diamino-1 -(2-thienyl)benzol∗2HCl /2,4,5,6-Tetraamino-pyrimindin-sulfat | 5-((2-Hydroxyethyl)amino)-1,3-benzodioxol∗HCl | grau-grün |
| **115** | 2,5-Diamino-1-(2-thienyl)benzol∗2HCl/2,4,5,6-Tetraamino-pyrimindin-sulfat | 5-Amino-2-methylphenol | violett |
| **116** | 2,5-Diamino-1-(2-thienyl)benzol∗2HCl/2,4,5,6-Tetraamino-pyrimindin-sulfat | 3-Amino-phenol | braun |
| **117** | 2,5-Diamino-1-(2-thienyl)benzol∗2HCl/2,4,5,6-Tetraamino-pyrimindin-sulfat | 1,3-Dihydroxybenzol | hellbraun |
| **118** | 2,5-Diamino-1-(2-thienyl)benzol∗2HCl/4,5-Diamino-1-(2-hydroxy-ethyl)-1H-pyrazol-sulfat | 2-Amino-4-(2'-hydroxyethyl)amino-anisolsulfat | dunkelviolett |
| **119** | 2,5-Diamino-1-(2-thienyl)benzol∗2HCl/4,5-Diamino-1-(2-hydroxy-ethyl)-1H-pyrazol-sulfat | 1,3-Dihydroxy-2-methyl-benzol | hellrot |
| **120** | 2,5-Diamino-1-(2-thienyl)benzol∗2HCl /4,5-Diamino-1-(2-hydroxyethyl)-1H-pyrazol-sulfat | 5-((2-Hydroxyethyl)amino)-1,3-benzodioxol*HCl | braun-rot |
| **121** | 2,5-Diamino-1-(2-thienyl)benzol∗2HCl /4,5-Diamino-1-(2-hydroxyethyl)-1H-pyrazol-sulfat | 5-Amino-2-methylphenol | rot |
| **122** | 2,5-Diamino-1-(2-thienyl)benzol∗2HCl/4,5-Diamino-1-(2-hydroxyethyl)-1H-pyrazol-sulfat | 3-Amino-phenol | rot |
| **123** | 2,5-Diamino-1-(2-thienyl)benzol∗2HCl/4,5-Diamino-1-(2-hydroxyethyl)-1H-pyrazol-sulfat | 1,3-Diamino-4-(2-hydroxyethoxy)benzol | violett |
| **124** | 2,5-Diamino-1-(2-thienyl)benzol∗2HCl /4,5-Diamino-1-(2-hydroxyethyl)-1H-pyrazol-sulfat | 1,3-Dihydroxybenzol | hellrot |

### Beispiel 125: Haarfärbemittel

Es werden Haarfärbelösungen der folgenden Zusammensetzung hergestellt:

| | |
|---|---|
| 0,160 g | 2,5-Diamino-1-(2-thienyl)benzol∗2HCl |
| 0,160 g | 1,4-Diamino-2-(2-hydroxyethyl)benzol∗sulfat |
| 0,137 g | 1,3-Dihydroxy-benzol |
| 0,100 g | 1,3-Dihydroxy-2-methylbenzol |
| 0,100 g | 2-Amino-5-methyl-phenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine blonde Färbung erhalten.

### Beispiel 126: Haarfärbemittel

Es werden Haarfärbelösungen der folgenden Zusammensetzung hergestellt:

| | |
|---|---|
| 0,320 g | 2,5-Diamino-1-(2-thienyl)benzol∗2HCl |
| 0,300 g | 5-Amino-2-methylphenol |
| 0,600 g | 4-Amino-3-methylphenol |
| 0,600 g | 4-Amino-phenol |
| 0,100 g | α-Naphtol |
| 0,200 g | 2-Chlor-6-(ethylamino)-4-nitrophenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine rote Färbung erhalten.

### Beispiel 127: Haarfärbemittel

Es werden Haarfärbelösungen der folgenden Zusammensetzung hergestellt:

| | |
|---|---|
| 0,320 g | 2,5-Diamino-1-(2-thienyl)benzol∗2HCl |
| 0,040 g | 5-Amino-2-methylphenol |
| 0,090 g | 2-Amino-4-(2'-hydroxyethyl)-amino-anisolsulfat |
| 0,030 g | 3-Aminophenol |
| 0,030 g | 1,3-Dihydroxy-benzol |
| 0,040 g | 1,3-Dihydroxy-2-methylbenzol |
| 0,100 g | 4-Amino-5-methylphenol |
| 0,200 g | 2-Amino-3-methylphenol |
| 0,100 g | 2-Amino-6-methylphenol-hydrochlorid |
| 0,010 g | 4-Chlor-N-(2-hydroxyethyl)-2-nitroanilin |
| 0,020 g | 2-Amino-4,6-dinitrophenol |
| 0,100 g | 2-Chlor-6-(ethylamino)-4-nitrophenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 128: Haarfärbemittel

Es werden Haarfärbelösungen der folgenden Zusammensetzung hergestellt:

| | |
|---|---|
| 0,320 g | 2,5-Diamino-1-(2-thienyl)benzol∗2HCl |
| 0,040 g | 5-Amino-2-methylphenol |
| 0,050 g | 1,3-Diamino-4-(2-hydroxyethoxy)-benzol |
| 0,030 g | 3-Aminophenol |
| 0,030 g | 1,3-Dihydroxy-benzol |
| 0,040 g | 1,3-Dihydroxy-2-methylbenzol |
| 0,100 g | 4-Amino-5-methylphenol |
| 0,200 g | 2-Amino-3-methylphenol |
| 0,100 g | 2-Amino-6-methylphenol-hydrochlorid |
| 0,010 g | 4-Chlor-N-(2-hydroxyethyl)-2-nitroanilin |
| 0,020 g | 2-Amino-4,6-dinitrophenol |
| 0,100 g | 2-Chlor-6-(ethylamino)-4-nitrophenol |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 129: Haarfärbemittel

Es werden Haarfärbelösungen der folgenden Zusammensetzung hergestellt:

| | |
|---|---|
| 0,220 g | 2,5-Diamino-1-(2-thienyl)benzol∗2HCl |
| 0,100 g | 1,4-Diamino-2-(2-hydroxyethyl)benzol∗sulfat |
| 0,020 g | 5-Amino-2-methylphenol |
| 0,010 g | 1,3-Diamino-4-(2-hydroxyethoxy)-benzol |
| 0,004 g | 2-Amino-4-(2'-hydroxyethyl)-amino-anisolsulfat |
| 0,020 g | 1,3-Dihydroxy-benzol |
| 0,040 g | 1,3-Dihydroxy-2-methylbenzol |
| 0,008 g | 4-Amino-3-methylphenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 130: Haarfärbemittel

Es werden Haarfärbelösungen der folgenden Zusammensetzung hergestellt:

| | |
|---|---|
| 0,220 g | 2,5-Diamino-1-(2-thienyl)benzol∗2HCl |
| 0,100 g | 4-Di-(2-hydroxyethyl)amino-anilin-sulfat |
| 0,020 g | 5-Amino-2-methylphenol |
| 0,010 g | 1,3-Diamino-4-(2-hydroxyethoxy)-benzol |
| 0,015 g | 2-Amino-4-(2'-hydroxyethyl)-amino-anisolsulfat |
| 0,020 g | 1,3-Dihydroxy-benzol |
| 0,040 g | 1,3-Dihydroxy-2-methylbenzol |
| 0,008 g | 4,5-Diamino-1-(2-hydroxyethyl)-1H-pyrazol-sulfat |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 131: Haarfärbemittel

Es werden Haarfärbelösungen der folgenden Zusammensetzung hergestellt

| | |
|---|---|
| 0,320 g | 2,5-Diamino-1-(2-thienyl)benzol∗2HCl |
| 0,020 g | 5-Amino-2-methylphenol |
| 0,010 g | 1,3-Diamino-4-(2-hydroxyethoxy)-benzol |
| 0,015 g | 2-Amino-4-(2'-hydroxyethyl)-amino-anisolsulfat |
| 0,020 g | 1,3-Dihydroxy-benzol |
| 0,040 g | 1,3-Dihydroxy-2-methylbenzol |
| 0,008 g | 4-Amino-2-(aminomethyl)phenol-dihydrochlorid |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

Alle Gewichtsangaben stellen, soweit nicht anders angegeben, Gewichtsprozente dar.

## Patentansprüche

1. Mittel zum oxidativen Färben von Keratinfasem auf der Basis einer Entwicklersubstanz-Kupplersubstanz-Kombination, **dadurch gekennzeichnet, daß** es als Entwicklersubstanz mindestens ein Diaminobenzol-Derivat der Formel (I) 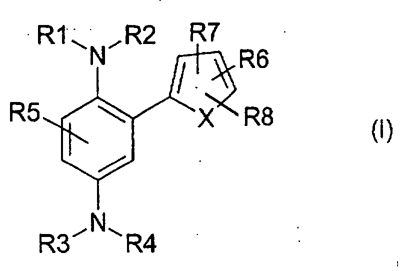 worin
**X** gleich Sauerstoff, Schwefel, Selen oder N-R9 ist;
**R1,R2,R3 und R4** unabhängig voneinander Wasserstoff, eine C₁-C₆-Alkylgruppe, eine C₁-C₄-Hydroxyalkylgruppe, eine C₂-C₄ Dihydroxyalkylgruppe oder eine C₁-C₄-Alkoxy-(C₁-C₂)-alkylgruppe darstellen oder R1und R2 beziehungsweise R3 und R4 einen viergliedrigen bis achtglied rigen aliphatischen Ring bilden, wobei mindestens 2 der Reste R1 bis R4 Wasserstoff darstellen;
**R5** gleich Wasserstoff, einem Halogenatom, einer C₁-C₄-Alkylgruppe, einer C₁-C₄ -Hydroxyalkylgruppe oder einer C₁-C₄-Alkoxygruppe ist;
**R6 und R8** unabhänging voneinander gleich Wasserstoff, einer Hydroxygruppe, einem Halogenatom, einer Cyanogruppe, einer C₁-C₄-Alkoxygruppe, einer C₁-C₆ -Alkylgruppe, einer C₁-C₄-Alkylthioethergruppe, einer Mercaptogruppe, einer Nitrogruppe, einer Aminogruppe, einer Alkylaminogruppe, einer Dialkylaminogruppe, einer -C(O)H-Gruppe, einer -C(O)CH₃-Gruppe, einer -C(O)CF₃-Gruppe, einer -Si(CH₃)₃-Gruppe, einer C₁-C₄-Hydroxyalkylgruppe, einer C₃-C₄-Dihydroxyalkylgruppe, einer -CH=CHR10-Gruppe, einer -(CH₂)ₚ-CO₂R11-Gruppe oder einer -(CH₂)ₚ-R12-Gruppe mit p=1,2,3 oder 4, einer -C(R13)=NR14-Gruppe oder einer C(R16)H-NR17R18-Gruppe sind;
**R7** gleich Wasserstoff, einem Halogenatom, einer C₁-C₆ Alkylgruppe oder einer -C(O)H-Gruppe ist;
**R9** gleich Wasserstoff, einer C₁-C₆-Alkylgruppe, einer C₁-C₄-Hydroxyalkylgruppe, einer Phenylgruppe oder einer Acetylgruppe ist;
**R10** gleich Wasserstoff, einer Hydroxygruppe, einer Nitrogruppe, einer Aminogruppe, einer -CO₂R11-Gruppe oder einer -C(O)CH₃-Gruppe ist;
**R11, R13 und R16** unabhänging voneinander gleich Wasserstoff oder einer C₁-C₄-Alkylgruppe sind;
**R12** gleich einer Aminogruppe oder einer Nitrilgruppe ist;
**R14, R17 und R18** unabhängig voneinander gleich Wasserstoff, einer Hydroxygruppe, einer C₁-C₄-Alkylgruppe, einer C₁-C₄-Hydroxyalkylgruppe, einer C₃-C₄-Dihydroxyalkylgruppe oder einem Rest der Formel (II) sind 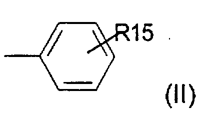 und
**R15** gleich Wasserstoff, einer Aminogruppe oder einer Hydroxygruppe ist, oder deren physiologisch verträgliche, wasserlösliche Salze.

2. Mittel nach Anspruch 1, **dadurch gekennzeichnet, daß** in der Formel (I) einer oder mehrere der Reste R5 bis R8 gleich Wasserstoff sind.

3. Mittel nach Anspruch 1 oder 2, **dadurch gekennzeichnet, daß** in der Formel (I) die Reste R1, R2, R3 und R4 gleich Wasserstoff sind.

4. Mittel nach einem der Ansprüche 1 bis 3, **dadurch gekennzeichnet, daß** in der Formel (I) R7 gleich Wasserstoff und R6 gleich Wasserstoff,-C(O)H, -C(O)CH₃, C₁-C₄-Alkyl oder C₁-C₄-Hydroxyalkyl ist.

5. Mittel nach Anspruch 4, **dadurch gekennzeichnet, daß** in der Formel (I) die Reste R7 und R6 gleich Wasserstoff sind.

6. Mittel nach einem der Ansprüche 1 bis 5, **dadurch gekennzeichnet, daß** es ausgewählt ist aus 2,5-Diamino-1-(2-thienyl)benzol, 2,5-Diamino-1-(2-furyl)benzol, 2,5-Diamino-1-(3-acetyl-2-thienyl)benzol, 2,5-Diamino-1-(3-chlor -2-thienyl)benzol, 2,5-Diamino-1-(1-H-pyrrol-2-yl)benzol, 2,5-Diamino-1-(3-methyl-2-thienyl)benzol, 2,5-Diamino-1-(5-methyl-2-thienyl)benzol, 2,5-Diamino-1-(3-nitro-2-thienyl)benzol, 2-Dimethylamino-5-amino-1-(2-thienyl)-benzol, 2-Pyrrolidino-5-amino-1-(2-thienyl)-benzol, 2-Di(2-Hydroxyethyl)amino-5-amino-1-(2-thienyl)-benzol, 2-(2-Hydroxyethyl)amino-5-amino-1-(2-thienyl)-benzol, 2-(2-Methoxyethyl)amino-5-amino-1-(2-thienyl)-benzol, 2-(2,3-Dihydroxypropyl)amino-5-amino-1-(2-thienyl)-benzol, 2,5-Diamino-4-methoxy-1-(2-thienyl)-benzol, 2,5-Diamino-4-methyl-1-(2-thienyl)-benzol, 2,5-Diamino-1-(3-chloro-2-thienyl)benzol, 2,5-Diamino-1-(1-methyl-pyrrol-2-yl)benzol und 2,5-Diamino-1-(3-formyl-2-thienyl)benzol.

7. Mittel nach einem der Ansprüche 1 bis 6, **dadurch gekennzeichnet, daß** das Diaminobenzol-Derivat der Formel (I) in einer Menge von 0,005 bis 20,0 Gewichtsprozent enthält.

8. Mittel nach einem der Ansprüche 1 bis 7, **dadurch gekennzeichnet, daß** die Kupplersubstanz ausgewählt ist aus 2,6-Diamino-pyridm, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxypyridin,3-Amino-6-methoxy-2-(methylamino)-pyridin, 2,6-Diamino-3,5-dimethoxy-pyridin, 3,5-Diamino-2,6-dimethoxy-pyridin, 1,3-Diaminobenzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-met)oxy-2-methyl-phenol, 5-Amino-4-ethoxy-2-methyl-phenol, 3-Amino-2,4-dichlorphenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl )amino]acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxynaphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxybenzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indolund 2,3-Indolindion.

9. Mittel nach einem der Ansprüche 1 bis 8, **dadurch gekennzeichnet, daß** es außer dem 1,4-Diaminobenzol-Derivat der Formel (I) zusätzlich mindestens eine weitere Entwicklersubstanz, welche-ausgewählt ist aus 1,4-Diaminobenzol, 2,5-Diaminotoluol, 2,5-Diaminophenylethylalkohol, 4-Aminophenol und seinen Derivaten, 4,5-Diaminopyrazolderivaten und Tetraaminopyrimidinen, enthält.

10. Mittel nach einem der Ansprüche 1 bis 9, **dadurch gekennzeichnet, daß** die Entwicklersubstanzen und Kupplersubstanzen, bezogen auf die Gesamtmenge des Oxidationsfärbemittel jeweils in einer Gesamtmenge von 0,005 bis 20 Gewichtsprozent enthalten sind.

11. Mittel nach einem der Ansprüche 1 bis 10, **dadurch gekennzeichnet, daß** es zusätzlich mindestens einen direktziehenden Farbstoff enthält.

12. Mittel nach einem der Ansprüche 1 bis 11, **dadurch gekennzeichnet, daß** es einen pH-Wert von 6,8 bis 11,5 aufweist.

13. Mittel nach einem der Ansprüche 1 bis 12, **dadurch gekennzeichnet, daß** es in Form einer wäßrigen oder wäßrigalkoholischen Lösung, einer Creme, eines Gels oder einer Emulsion vorliegt.

14. Mittel nach einem der Ansprüche 1 bis 13, **dadurch gekennzeichnet, daß** es ein Haarfärbemittel ist.

15. Diaminobenzol-Derivat der Formel (I) 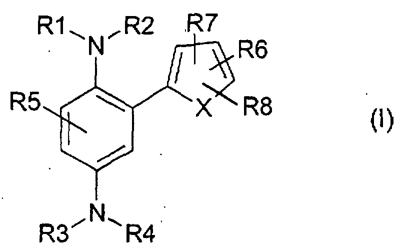 worin
**X** gleich Sauerstoff, Schwefel, Selen oder N-R9 ist;
**R1,R2,R3 und R4** unabhängig voneinander Wasserstoff, eine C₁-C₆-Alkylgruppe, eine C₁-C₄-Hydroxyalkylgruppe, eine C₂-C₄ Dihydroxyalkylgruppe oder eine C₁-C₄-Alkoxy-(C₁-C₂)-alkylgruppe darstellen oder R1und R2 beziehungsweise R3 und R4 einen viergliedrigen bis achtgliedrigen aliphatischen Ring bilden, wobei mindestens 2 der Reste R1 bis R4 Wasserstoff darstellen;
**R5** gleich Wasserstoff, einem Halogenatom, einer C₁-C₄-Alkylgruppe, einer C₁-C₄ -Hydroxyalkylgruppe oder einer C₁-C₄-Alkoxygruppe ist;
**R6 und R8** unabhänging voneinander gleich Wasserstoff, einer Hydroxygruppe, einem Halogenatom, einer Cyanogruppe, einer C₁-C₄-Alkoxygruppe, einer C₁-C₆ -Alkylgruppe, einer C₁-C₄-Alkylthioethergruppe, einer Mercaptogruppe, einer Nitrogruppe, einer Aminogruppe, einer Alkylaminogruppe, einer Dialkylaminogruppe, einer -C(O)H-Gruppe, einer -C(O)CH₃-Gruppe, einer -C(O)CF₃-Gruppe, einer -Si(CH₃)₃-Gruppe, einer C₁-C₄-Hydroxyalkylgruppe, einer C₃-C₄-Dihydroxyalkylgruppe, einer -CH=CHR10-Gruppe, einer -(CH₂)ₚ-CO₂R11-Gruppe oder einer -(CH₂)ₚ-R12-Gruppe mit p=1,2,3 oder 4, einer -C(R13)=NR14-Gruppe oder einer C(R16)H-NR17R18-Gruppe sind;
**R7** gleich Wasserstoff, einem Halogenatom, einer C₁-C₆ Alkylgruppe oder einer -C(O)H-Gruppe ist;
**R9** gleich Wasserstoff, einer C₁-C₆-Alkylgruppe, einer C₁-C₄-Hydroxyalkylgruppe, einer Phenylgruppe oder einer Acetylgruppe-ist;
**R10** gleich Wasserstoff, einer Hydroxygruppe, einer Nitrogruppe, einer Aminogruppe, einer -CO₂R11-Gruppe oder einer -C(O)CH₃-Gruppe ist;
**R11, R13 und R16** unabhänging voneinander gleich Wasserstoff oder einer C₁-C₄-Alkylgruppe sind;
**R12** gleich einer Aminogruppe oder einer Nitrilgruppe ist;
**R14, R17 und R18** unabhängig voneinander gleich Wasserstoff, einer Hydroxygruppe, einer C₁-C₄-Alkylgruppe, einer C₁-C₄-Hydroxyalkylgruppe, einer C₃-C₄-Dihydroxyalkylgruppe oder einem Rest der Formel (II) sind 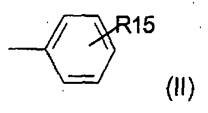 und
**R15** gleich Wasserstoff, einer Aminogruppe oder einer Hydroxygruppe ist, unter der Bedingung, daß, wenn gilt X= Sauerstoff und **R1= R2=** Methyl, mindestens einer der Reste R3 bis R8 verschieden von Wasserstoff ist, oder deren physiologisch verträgliche, wasserlösliche Salze.

16. p-Diaminobenzol-Derivat nach Anspruch 15, **dadurch gekennzeichnet, daß** (i) eine oder mehrere der Restgruppen R1, R2, R3, R4, R5, R6, R7 und R8 gleich Wasserstoff sind und/oder (ii) R1, R2, R3 und R4 gleichzeitig Wasserstoff bedeuten und/oder (iii) R7 gleich Wasserstoff und R6 gleich Wasserstoff, -C(O)H, -C(O)CH₃, C₁-C₄-Alkyl oder C₁-C₄-Hydroxyalkyl ist.

17. p-Diaminobenzol-Derivat nach Anspruch 15 oder 16, **dadurch gekennzeichnet, daß** es ausgewählt ist aus 2,5-Diamino-1-(2-thienyl)benzol, 2,5-Diamino-1-(2-furyl)benzol, 2,5-Diamino-1-(3-acetyl-2-thienyl)benzol, 2,5-Diamino-1-(3-chlor -2-thienyl)benzol, 2,5-Diamino-1-(1-H-pyrrol-2-yl)benzol, 2,5-Diamino-1-(3-methyl-2-thienyl)benzol, 2,5-Diamino-1-(5-methyl-2-thienyl)benzol, 2,5-Diamino-1-(3-nitro-2-thienyl)benzol, 2-Dimethylamino-5-amino-1-(2-thienyl)-benzol, 2-Pyrrolidino-5-amino-1-(2-thienyl)-benzol, 2-Di(2-Hydroxyethyl)amino-5-amino-1-(2-thienyl)-benzol, 2-(2-Hydroxy-ethyl)amino-5-amino-1-(2-thienyl)-benzol, 2-(2-Methoxyethyl)amino-5-amino-1-(2-thienyl)-benzol, 2-(2,3-Dihydroxypropyl)amino-5-amino-1-(2-thienyl)-benzol, 2,5-Diamino-4-methoxy-1-(2-thienyl)-benzol, 2,5-Diamino-4-methyl-1-(2-thienyl)-benzol, 2,5-Diamino-1-(3-chloro-2-thienyl)benzol, 2,5-Diamino-1-(1-methyl-pyrrol-2-yl)benzol und 2,5-Diamino-1-(3-formyl-2-thienyl)benzol.

## Claims

1. Agent for the oxidative colouring of keratin fibres based on a developer substance-coupler substance combination, **characterized in that** it comprises, as developer substance, at least one diaminobenzene derivative of the formula (I) 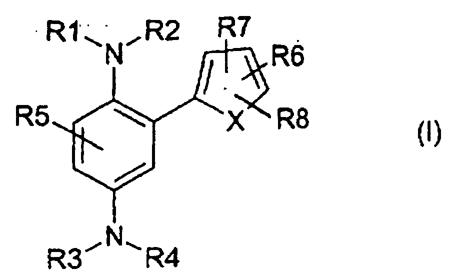 in which
**X** is oxygen, sulphur, selenium or N-R9;
**R1, R2, R3 and R4,** independently of one another, are hydrogen, a C₁-C₆-alkyl group, a C₁-C₄-hydroxyalkyl group, a C₂-C₄-dihydroxyalkyl group or a C₁-C₄-alkoxy-(C₁-C₂)-alkyl group, or R1 and R2, or R3 and R4 form a 4-membered to 8-membered aliphatic ring, where at least two of the radicals R1 to R4 are hydrogen;
**R5** is hydrogen, a halogen atom, a C₁-C₄-alkyl group, a C₁-C₄-hydroxyalkyl group or a C₁-C₄-alkoxy group;
**R6 and R8,** independently of one another, are hydrogen, a hydroxyl group, a halogen atom, a cyano group, a C₁-C₄-alkoxy group, a C₁-C₆-alkyl group, a C₁-C₄-alkyl thioether group, a mercapto group, a nitro group, an amino group, an alkylamino group, a dialkylamino group, a -C(O)H group, a -C(O)CH₃ group, a -C(O)CF₃ group, a -Si(CH₃)₃ group, a C₁-C₄-hydroxyalkyl group, a C₃-C₄-dihydroxyalkyl group, a -CH=CHR10 group, a -(CH₂)ₚ-CO₂R11 group or a -(CH₂)ₚ-R12 group where p = 1, 2, 3 or 4, a -C(R13)=NR14 group or a C(R16)H-NR17R18 group;
**R7** is hydrogen, a halogen atom, a C₁-C₆-alkyl group or a -C(O)H group;
**R9** is hydrogen, a C₁-C₆-alkyl group, a C₁-C₄-hydroxyalkyl group, a phenyl group or an acetyl group;
**R10** is hydrogen, a hydroxyl group, a nitro group, an amino group, a -CO₂R11 group or a -C(O)CH₃ group;
**R11, R13 and R16,** independently of one another, are hydrogen or a C₁-C₄-alkyl group;
**R12** is an amino group or a nitrile group;
**R14, R17 and R18,** independently of one another are hydrogen, a hyroxyl group, a C₁-C₄-alkyl group, a C₁-C₄-hydroxyalkyl group, a C₃-C₄-dihydroxyalkyl group or a radical of the formula (II) 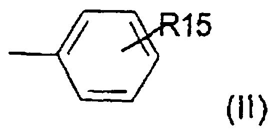 and
**R15** is hydrogen, an amino group or a hydroxyl group,
or physiologically compatible, water-soluble salts thereof.

2. Agent according to Claim 1, **characterized in that**, in the formula (I), one or more of the radicals R5 to R8 are hydrogen.

3. Agent according to Claim 1 or 2, **characterized in that**, in the formula (I), the radicals R1, R2, R3 and R4 are hydrogen.

4. Agent according to one of Claims 1 to 3, **characterized in that**, the formula (I), R7 is hydrogen and R6 is hydrogen, C(O)H, -C(O)CH₃, C₁-C₄-alkyl or C₁-C₄-hydroxyalkyl.

5. Agent according to Claim 4, **characterized in that**, in the formula (I), the radicals R7 and R6 are hydrogen.

6. Agent according to one of Claims 1 to 5, **characterized in that** it is chosen from 2,5-diamino-1-(2-thienyl)benzene, 2,5-diamino-1-(2-furyl)benzene, 2,5-diamino-1-(3-acetyl-2-thienyl)benzene, 2,5-diamino-1-(3-chloro-2-thienyl)benzene, 2,5-diamino-1-(1H-pyrrol-2-yl)benzene, 2,5-diamino-1-(3-methyl-2-thienyl)benzene, 2,5-diamino-1-(5-methyl-2-thienyl)benzene, 2,5-diamino-1-(3-nitro-2-thienyl)benzene, 2-dimethylamino-5-amino-1-(2-thienyl)benzene, 2-pyrrolidino-5-amino-1-(2-thienyl)benzene, 2-di(2-hydroxyethyl)amino-5-amino-1-(2-thienyl)benzene, 2-(2-hydroxyethyl)amino-5-amino-1-(2-thienyl)benzene, 2-(2-methoxyethyl)amino-5-amino-1-(2-thienyl)benzene, 2-(2,3-dihydroxypropyl)amino-5-amino-1-(2-thienyl)benzene, 2,5-diamino-4-methoxy-1-(2-thienyl)benzene, 2,5-diamino-4-methyl-1-(2-thienyl)benzene, 2,5-diamino-1-(3-chloro-2-thienyl)benzene, 2,5-diamino-1-(1-methylpyrrol-2-yl)benzene and 2,5-daimino-1-(3-formyl-2-thienyl)benzene.

7. Agent according to one of Claims 1 to 6, **characterized in that** it comprises the diaminobenzene derivative of the formula (I) in an amount of from 0.005 to 20.0 per cent by weight.

8. Agent according to one of Claims 1 to 7, **characterized in that** the coupler substance is chosen from 2, 6-diaminopyridine, 2-amino-4-[2-(hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-2-methylphenol, 3-[(2-hydroxyethyl)amino]phenol, 3-[(2-methoxyethyl)amino]phenol, 5-amino-2-ethylphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 5-amino-4-chloro-2-methylphenol, 1-naphthol, 1,5-dihydroxynaphthalene, 1,7-dihydroxynaphthalene, 2,3-dihydroxynaphthalene, 2,7-dihydroxynaphthalene, 2-methyl-1-napthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole,6-hydroxyindole, 7-hydroxyindole and 2,3-indolinedione.

9. Agent according to one of Claims 1 to 8, **characterized in that**, apart from the 1,4-diaminobenzene derivative of the formula (I), it additionally comprises at least one further developer substance which is chosen from 1,4-diaminobenzene, 2,5-diaminotoluene, 2,5-diaminophenylethyl alcohol, 4-aminophenol and its derivatives, 4,5-diaminopyrazole derivatives and tetraaminopyrimidines.

10. Agent according to one of Claims 1 to 9, **characterized in that** the developer substances and coupler substances, based on the total amount of the oxidation colorant, are present in each case in a total amount of from 0.005 to 20 per cent by weight.

11. Agent according to one of Claims 1 to 10, **characterized in that** it additionally comprises at least one substantive dye.

12. Agent according to one of Claims 1 to 11, **characterized in that** it has a pH of from 6.8 to 11.5.

13. Agent according to one of Claims 1 to 12, **characterized in that** it is in the form of an aqueous or aqueous-alcoholic solution, a cream, a gel or an emulsion.

14. Agent according to one of Claims 1 to 13, **characterized in that** it is a hair colorant.

15. Diaminobenzene derivative of the formula (I) 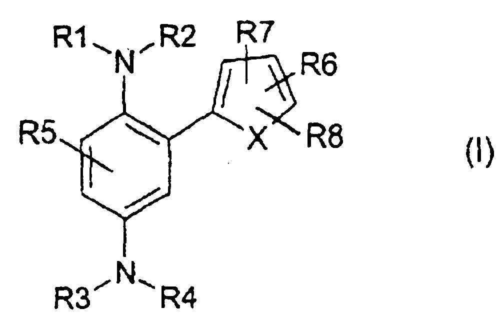 in which
**X** is oxygen, sulphur, selenium or N-R9;
**R1, R2, R3 and R4,** independently of one another, are hydrogen, a C₁-C₆-alkyl group, a C₁-C₄-hydroxyalkyl group, a C₂-C₄-dihydroxyalkyl group or a C₁-C₄-alkoxy(C₁-C₂)-alkyl group, or R1 and R2, or R3 and R4 form a 4-membered to 8-membered aliphatic ring, where at least two of the radicals R1 to R4 are hydrogen;
**R5** is hydrogen, a halogen atom, a C1-C4-alkyl group, a C₁-C₄-hydroxyalkyl group or a C₁-C₄-alkoxy group;
**R6 and R8,** independently of one another, are hydrogen, a hydroxyl group, a hydrogen atom, a cyano group, a C₁-C₄-alkoxy group, a C₁-C₆-alkyl group, a C₁-C₄-alkyl thioether group, a mercapto group, a nitro group, an amino group, an alkylamino group, a dialkylamino group, a -C(O)H group, a -C(O)CH₃ group, a -C(O)CF₃ group, a -Si(CH₃)₃ group, a C₁-C₄-hydroxyalkyl group, a C₃-C₄-dihydroxyalkyl group, a -CH=CHR10 group, a -(CH₂)ₚ-CO₂R11 group or a -(CH₂)ₚ-R12 group where p = 1, 2, 3 or 4, a -C(R13)=NR14 group or a C(R16)H-NR17R18 group;
**R7** is hydrogen, a halogen atom, a C₁-C₆-alkyl group or a -C(O)H group;
**R9** is hydrogen, a C₁-C₆-alkyl group, a C₁-C₄-hydroxyalkyl group, a phenyl group or an acetyl group;
**R10** is hydrogen, a hydroxyl group, a nitro group, an amino group, a -CO₂R11 group or a -C(O)CH₃ group;
**R11, R13 and R16,** independently of one another, are hydrogen or a C₁-C₄-alkyl group;
**R12** is an amino group or a nitrile group;
**R14, R17 and R18,** independently of one another are hydrogen, a hydroxyl group, a C₁-C₄-alkyl group, a C₁-C₄-hydroxyalkyl group, a C₃-C₄-dihydroxyalkyl group or a radical of the formula (II) 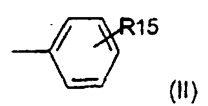 and
**R15** is hydrogen, an amino group or a hydroxyl group,
on condition that, if **X =** oxygen and **R1 = R2 =** methyl, at least one of the radicals **R3** to **R8** is different to hydrogen, or physiologically compatible, water-soluble salts thereof.

16. p-Diaminobenzene derivative according to Claim 15, **characterized in that** (i) is one or more of the radical groups R1, R2, R3, R4, R5, R6, R7 and R8 are hydrogen and/or (ii) R1, R2, R3 and R4 are hydrogen at the same time and/or (iii) R7 is hydrogen and R6 is hydrogen, -C(O)H, -C(O)CH₃, C₁-C₄-alkyl or C₁-C₄-hydroxyalkyl.

17. p-Diaminobenzene derivative according to Claim 15 or 16, **characterized in that** it is chosen from 2,5-diamino-1-(2-thienyl)benzene, 2,5-diamino-1-(2-furyl)benzene, 2,5-diamino-1-(3-acetyl-2-thienyl)benzene, 2,5-diamino-1-(3-chloro-2-thienyl)benzene, 2,5-diamino-1-(1H-pyrrol-2-yl)benzene, 2,5-diamino-1-(3-methyl-2-thienyl)benzene, 2,5-diamino-1-(5-methyl-2-thienyl)benzene, 2,5-diamino-1-(3-nitro-2-thienyl)benzene, 2-dimethylamino-5-amino-1-(2-thienyl)benzene, 2-pyrrolidino-5-amino-1-(2-thienyl)benzene, 2-di(2-hydroxyethyl)amino-5-amino-1-(2-thienyl)benzene, 2-(2-hydroxyethyl)amino-5-amino-1-(2-thienyl)benzene, 2-(2-methoxyethyl)amino-5-amino-1-(2-thienyl)benzene, 2-(2,3-dihydroxypropyl)amino-5-amino-1-(2-thienyl)benzene, 2,5-diamino-4-methoxy-1-(2-thienyl)benzene, 2,5-diamino-4-methyl-1-(2-thienyl)benzene, 2,5-diamino-1-(3-chloro-2-thienyl)benzene, 2,5-diamino-1-(1-methylpyrrol-2-yl)benzene and 2,5-diamino-1-(3-formyl-2-thienyl)benzene.

## Revendications

1. Agent pour la teinture par oxydation de fibres de kératine, à base d'une association de substance de type copulant - substance de type développeur, **caractérisé en ce qu'**il contient comme substance de type développeur au moins un dérivé de diaminobenzène de formule (I) 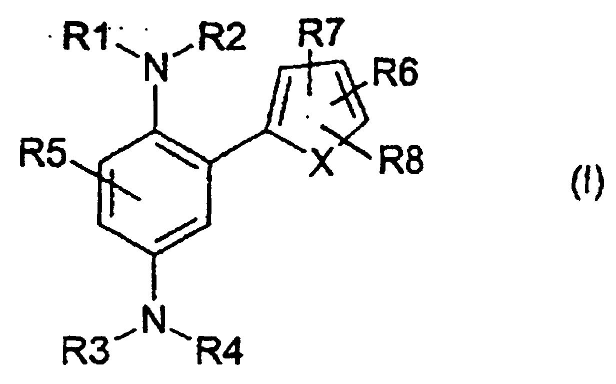 dans laquelle
X est un atome d'oxygène, de soufre ou de sélénium, ou N-R9 ;
R1, R2, R3 et R4 représentent, indépendamment les uns des autres, un atome d'hydrogène, un groupe alkyle en C₁-C₆, un groupe hydroxyalkyle en C₁-C₄, un groupe dihydroxyalkyle en C₂-C₄ ou un groupe alcoxy(C₁-C₄)-alkyle(C₁-C₂), ou R1 et R2 ou, respectivement, R3 et R4, forment un cycle aliphatique à 4-8 chaînons, au moins deux des radicaux R1 à R4 représentant des atomes d'hydrogène ;
R5 représente un atome d'hydrogène ou d'halogène, ou un groupe alkyle en C₁-C₄, un groupe hydroxyalkyle en C₁-C₄ ou un groupe alcoxy en C₁-C₄ ;
R6 et R8 représentent, indépendamment l'un de l'autre, un atome d'hydrogène, un groupe hydroxy, un atome d'halogène, un groupe cyano, un groupe alcoxy en C₁-C₄, un groupe alkyle en C₁-C₆, un groupe alkyl (C₁-C₄)thioéther, un groupe mercapto, un groupe nitro, un groupe amino, un groupe alkylamino, un groupe dialkylamino, un groupe -C(O)H, un groupe -C(O)CH₃, un groupe -C(O)CF₃, un groupe -Si(CH₃)₃, un groupe hydroxyalkyle en C₁-C₄, un groupe dihydroxyalkyle en C₃-C₄, un groupe -CH=CHR10, un groupe -(CH₂)ₚ-CO₂R11 ou un groupe -(CH₂)ₚ-R12 où p = 1, 2, 3 ou 4, un groupe -C(R13)=NR14 ou un groupe C(R16)H-NR17R18 ;
R7 représente un atome d'hydrogène, un atome d'halogène, un groupe alkyle en C₁-C₆ ou un groupe -C(O)H ;
R9 représente un atome d'hydrogène, un groupe alkyle en C₁-C₆, un groupe hydroxyalkyle en C₁-C₄, un groupe phényle ou un groupe acétyle ;
R10 représente un atome d'hydrogène, un groupe hydroxy, un groupe nitro, un groupe amino, un groupe -CO₂R11 ou un groupe -C(O)CH₃ ;
R11, R13 et R16 représentent, indépendamment les uns des autres, un atome d'hydrogène ou un groupe alkyle en C₁-C₄ ;
R12 représente un groupe amino ou un groupe nitrilo ;
R14, R17 et R18 représentent, indépendamment les uns des autres, un atome d'hydrogène, un groupe hydroxy, un groupe alkyle en C₁-C₄, un groupe hydroxyalkyle en C₁-C₄, un groupe dihydroxyalkyle en C₃-C₄ ou un radical de formule (II) 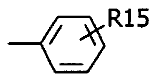 et
R15 représente un atome d'hydrogène, un groupe amino ou un groupe hydroxy,
ou des sels hydrosolubles, physiologiquement acceptables, de tels composés.

2. Agent selon la revendication 1, **caractérisé en ce que** dans la formule (I) un ou plusieurs des radicaux R5 à R8 représente(nt) un(des) atome(s) d'hydrogène.

3. Agent selon la revendication 1 ou 2, **caractérisé en ce que** dans la formule (I) les radicaux R1, R2, R3 et R4 représentent des atomes d'hydrogène.

4. Agent selon l'une quelconque des revendications 1 à 3, **caractérisé en ce que** dans la formule (I) R7 représente un atome d'hydrogène et R6 représente un atome d'hydrogène ou un groupe -C(O)H, -C(O)CH₃, alkyle en C₁-C₄ ou hydroxyalkyle en C₁-C₄.

5. Agent selon la revendication 4, **caractérisé en ce que** dans la formule (I) les radicaux R7 et R6 représentent des atomes d'hydrogène.

6. Agent selon l'une quelconque des revendications 1 à 5, **caractérisé en ce qu'**il est choisi parmi le 2,5-diamino-1-(2-thiényl)benzène, le 2,5-diamino-1-(2-furyl)benzène, le 2,5-diamino-1-(3-acétyl-2-thiényl)benzène, le 2,5-diamino-1-(3-chloro-2-thiényl)benzène, le 2,5-diamino-1-(1-H-pyrrol-2-yl)benzène, le 2,5-diamino-1-(3-méthyl-2-thiényl)benzène, le 2,5-diamino-1-(5-méthyl-2-thiényl)benzène, le 2,5-diamino-1-(3-nitro-2-thiényl)benzène, le 2-diméthylamino-5-amino-1-(2-thiényl)benzène, le 2-pyrrolidino-5-amino-1-(2-thiényl)benzène, le 2-di(2-hydroxyéthyl)amino-5-amino-1-(2-thiényl)benzène, le 2-(2-hydroxyéthyl)amino-5-amino-1-(2-thiényl)benzène, le 2-(2-méthoxyéthyl)amino-5-amino-1-(2-thiényl)benzène, le 2-(2,3-dihydroxypropyl)amino-5-amino-1-(2-thiényl)benzène, le 2,5-diamino-4-méthoxy-1-(2-thiényl)benzène, le 2,5-diamino-4-méthyl-1-(2-thiényl)benzène, le 2,5-diamino-1-(3-chloro-2-thiényl)benzène, le 2,5-diamino-1-(1-méthyl-pyrrol-2-yl)benzène et le 2,5-diamino-1-(3-formyl-2-thiényl)benzène.

7. Agent selon l'une quelconque des revendications 1 à 6, **caractérisé en ce qu'**il contient le dérivé de diaminobenzène de formule (I) en une proportion de 0,005 à 20,0 % en poids.

8. Agent selon l'une quelconque des revendications 1 à 7, **caractérisé en ce que** la substance de type copulant est choisie parmi la 2,6-diaminopyridine, le 2-amino-4[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthl)amino]-1,5-diméthoxybenzène, la 2,3-diamino-6-méthoxypyridine, la 3-amino-6-méthoxy-2-(méthylamino)pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]aniline, le 4-amino-2-di[(2-hydroxyéthyl)amino]-1-éthoxybenzène, le 5-méthyl-2-(1-méthyléthyl)-phénol, la 3-[(2-hydroxyéthyl)amino]aniline, la 3-[(2-aminoéthyl)amino]aniline, le 1,3-di(2,4-diaminophénoxypropane, le di(2,4-diaminophénoxy)méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphenol, le 3-amino-2,4-dichlorophénol, le 5-amino-2,4-dichlorophénol, le 3-amino-2-méthylphénol, le 3-amino-2chloro-6-méthylphenol, le 3-aminophénol, 2-[(3-hydroxyphényl)amino]acétamide, le 5-[(2-hydroxyéthyl)amino]-2-méthylphénol, le 3-[(2-hydroxyéthyl)amino]phénol, 3-[(2-méthoxyéthyl)amino]phénol, le 5-amino-2-éthylphénol, le 2-(4-amino-2-hydroxyphénoxy)éthanol, le 5-[(3-hydroxypropyl)amino]-2-méthylphénol, le 3-[(2,3-dihydrpxypropyl)amino]-2-méthylphénol, le 3-[(2-hydroxyéthyl)amino]-2-méthylphénol, la 2-amino-3-hydroxypyridine, le 5-amino-4-chloro-2-méthylphénol, le 1-naphtol, le 1,5-dihydroxynaphthalène, 1,7-dihydroxynaphthalène, le 2,3-dihydroxynaphthalène, le 2,7-dihydroxynaphthalène, l'acétate de 2-méthyl-1-naphtol, le 1,3-dihydroxybenzène, le 1-chloro-2,4-dihydroxybenzène, le 2-chloro-1,3-dihydroxybenzène, 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione.

9. Agent selon l'une quelconque des revendications 1 à 8, **caractérisé en ce qu'**en plus du dérivé de 1,4-diaminobenzène de formule (I), il contient encore au moins une autre substance de type développeur qui est choisie parmi le 1,4-diaminobenzène, le 2,5-diaminotoluène, l'alcool 2,5-diaminophényléthylique, le 4-aminophénol et ses dérivés, des dérivés de 4,5-diaminopyrazole et les tétraaminopyrimidines.

10. Agent selon l'une quelconque des revendications 1 à 9, **caractérisé en ce que** les substances de type développeur et les substances de type copulant sont contenues respectivement en une proportion totale de 0,005 à 20 % en poids, par rapport à la quantité totale du colorant d'oxydation.

11. Agent selon l'une quelconque des revendications 1 à 10, **caractérisé en ce qu'**il contient en outre au moins un colorant direct.

12. Agent selon l'une quelconque des revendications 1 à 11, **caractérisé en ce qu'**il présente un pH de 6,8 à 11,5.

13. Agent selon l'une quelconque des revendications 1 à 12, **caractérisé en ce qu'**il se présente sous forme d'une solution aqueuse ou aqueuse-alcoolique, d'une crème, d'un gel ou d'une émulsion.

14. Agent selon l'une quelconque des revendications 1 à 13, **caractérisé en ce qu'**il est un agent de teinture pour cheveux.

15. Dérivé de diaminobenzène de formule (I) 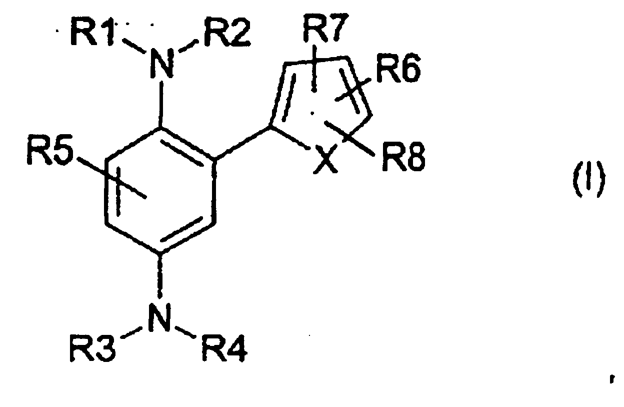 dans laquelle
X est un atome d'oxygène, de soufre ou de sélénium, ou N-R9 ;
R1, R2, R3 et R4 représentent, indépendamment les uns des autres, un atome d'hydrogène, un groupe alkyle en C₁-C₆, un groupe hydroxyalkyle en C₁-C₄, un groupe dihydroxyalkyle en C₂-C₄ ou un groupe alcoxy(C₁-C₄)-alkyle(C₁-C₂), ou R1 et R2 ou, respectivement, R3 et R4, forment un cycle aliphatique à 4-8 chaînons, au moins deux des radicaux R1 à R4 représentant des atomes d'hydrogène ;
R5 représente un atome d'hydrogène ou d'halogène, ou un groupe alkyle en C₁-C₄, un groupe hydroxyalkyle en C₁-C₄ ou un groupe alcoxy en C₁-C₄ ;
R6 et R8 représentent, indépendamment l'un de l'autre, un atome d'hydrogène, un groupe hydroxy, un atome d'halogène, un groupe cyano, un groupe alcoxy en C₁-C₄, un groupe alkyle en C₁-C₆, un groupe alkyl(C₁-C₄)thioéther, un groupe mercapto, un groupe nitro, un groupe amino, un groupe alkylamino, un groupe dialkylamino, un groupe -C(O)H, un groupe -C(O)CH₃, un groupe -C(O)CF₃, un groupe -Si(CH₃)₃, un groupe hydroxyalkyle en C₁-C₄, un groupe dihydroxyalkyle en C₃-C₄, un groupe -CH=CHR10, un groupe -(CH₂)ₚ-CO₂R11 ou un groupe -(CH₂)ₚ-R12 où p = 1, 2, 3 ou 4, un groupe -C(R13)=NR14 ou un groupe C(R16)H-NR17R18 ;
R7 représente un atome d'hydrogène, un atome d'halogène, un groupe alkyle en C₁-C₆ ou un groupe -C(O)H ;
R9 représente un atome d'hydrogène, un groupe alkyle en C₁-C₆, un groupe hydroxyalkyle en C₁-C₄, un groupe phényle ou un groupe acétyle ;
R10 représente un atome d'hydrogène, un groupe hydroxy, un groupe nitro, un groupe amino, un groupe -CO₂R11 ou un groupe -C(O)CH₃ ;
R11, R13 et R16 représentent, indépendamment les uns des autres, un atome d'hydrogène ou un groupe alkyle en C₁-C₄ ;
R12 représente un groupe amino ou un groupe nitrilo ;
R14, R17 et R18 représentent, indépendamment les uns des autres, un atome d'hydrogène, un groupe hydroxy, un groupe alkyle en C₁-C₄, un groupe hydroxyalkyle en C₁-C₄, un groupe dihydroxyalkyle en C₃-C₄ ou un radical de formule (II) 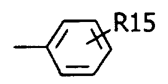 et
R15 représente un atome d'hydrogène, un groupe amino ou un groupe hydroxy,
avec la condition que lorsque X est un atome d'oxygène et R1=R2= le groupe méthyle, au moins l'un des radicaux R3 à R8 est différent d'un atome d'hydrogène,
ou sels hydrosolubles, physiologiquement acceptables, de tels composés.

16. Dérivé de p-diaminobenzène selon la revendication 15, **caractérisé en ce que** (I) un ou plusieurs des radicaux R1, R2, R3, R4, R5, R6, R7 et R8 représente(nt) un(des) atome(s) d'hydrogène et/ou (II) R1, R2, R3 et R4 représentent simultanément des atomes d'hydrogène et/ou (III) R7 représente un atome d'hydrogène et R6 représente un atome d'hydrogène ou un groupe -C(O)H, -C(O)CH₃, alkyle en C₁-C₄ ou hydroxyalkyle en C₁-C₄.

17. Dérivé de p-diaminobenzène selon la revendication 15 ou 16, **caractérisé en ce qu'**il est choisi parmi le 2,5-diamino-1-(2-thiényl)benzène, le 2,5-diamino-1-(2-furyl)benzène, le 2,5-diamino-1-(3-acétyl-2-thiényl)benzène, le 2,5-diamino-1-(3-chloro-2-thiényl)benzène, le 2,5-diamino-1-(1-H-pyrrol-2-yl)benzène, le 2,5-diamino-1-(3-méthyl-2-thiényl)benzène, le 2,5-diamino-1-(5-méthyl-2-thiényl)benzène, le 2,5-diamino-1-(3-nitro-2-thiényl)benzène, le 2-diméthylamino-5-amino-1-(2-thiényl)benzène, le 2-pyrrolidino-5-amino-1-(2-thiényl)benzène, le 2-di(2-hydroxyéthyl)amino-5-amino-1-(2-thiényl)benzène, le 2-(2-hydroxyéthyl)amino-5-amino-1-(2-thiényl)benzène, le 2-(2-méthoxyéthyl)amino-5-amino-1-(2-thiényl)benzène, le 2-(2,3-dihydroxypropyl)amino-5-amino-1-(2-thiényl)benzène, le 2,5-diamino-4-méthoxy-1-(2-thiényl)benzène, le 2,5-diamino-4-méthyl-1-(2-thiényl)benzène, le 2,5-diamino-1-(3-chloro-2-thiényl)benzène, le 2,5-diamino-1-(1-méthyl-pyrrol-2-yl)benzène et le 2,5-diamino-1-(3-formyl-2-thiényl)benzène.
